# EUROPEAN PATENT SPECIFICATION

(11) **EP 1 740 204 B1**
(45) Date of publication and mention of the grant of the patent: **14.03.2018**
(21) Application number: 05715146.6
(22) Date of filing: 01.04.2005
(51) Int. Cl.: A61K 38/47, A61P 25/00, A61K 38/46

(54) **MEDICINAL USE OF ALPHA-MANNOSIDASE**
MEDIZINISCHE ANWENDUNG DER ALPHA-MANNOSIDASE
UTILISATION MEDICALE DE L'ALPHA-MANNOSIDASE

(30) Priority: 01.04.2004 DK 200400528; 01.04.2004 US 558108 P
(43) Date of publication of application: 10.01.2007
(73) Proprietor: Chiesi Farmaceutici S.p.A., 43122 Parma (IT)
(72) Inventor: ANDERSSON, Claes, S-187 43 Täby (SE); FOGH, Jens, DK-3540 Lynge (DK); WEIGELT, Cecilia, S-115 22 Stockholm (SE); ROCES, Diego, Prieto, DE-37075 Göttingen (DE); VON FIGURA, Kurt, DE-37085 Göttingen (DE); IRANI, Meher, Seattle, WA 98125 (US)
(74) Representative: Plougmann Vingtoft a/s
(86) International application number: PCT/DK2005/000228
(87) International publication number: WO 2005/094874

(56) References cited:
- WO-A-00/73482
- WO-A-02/099092
- BERG THOMAS ET AL: "Purification and characterization of recombinant human lysosomal alpha-mannosidase" MOLECULAR GENETICS AND METABOLISM, ACADEMIC PRESS, SAN DIEGO, CA, US, vol. 73, no. 1, 24 April 2001 (2001-04-24), pages 18-29, XP002182076 ISSN: 1096-7192
- BERG^A T ET AL: "alpha-Mannosidosis in the guinea pig: cloning of the lysosomal alpha-mannosidase cDNA and identification of a missense mutation causing alpha-mannosidosis" BIOCHIMICA ET BIOPHYSICA ACTA. MOLECULAR BASIS OF DISEASE, AMSTERDAM, NL, vol. 1586, no. 2, 16 March 2002 (2002-03-16), pages 169-176, XP004349023 ISSN: 0925-4439
- MERKLE R K ET AL: "Cloning, expression, purification, and characterization of the murine lysosomal acid alpha-mannosidase" BBA - GENERAL SUBJECTS, ELSEVIER SCIENCE PUBLISHERS, NL, vol. 1336, no. 2, 27 August 1997 (1997-08-27), pages 132-146, XP004276600 ISSN: 0304-4165
- GONZALEZ D S ET AL: "Isolation and characterization of the gene encoding the mouse broad specificity lysosomal alpha-mannosidase" BIOCHIMICA ET BIOPHYSICA ACTA . GENE STRUCTURE AND EXPRESSION, ELSEVIER, AMSTERDAM, NL, vol. 1445, no. 1, 14 April 1999 (1999-04-14), pages 177-183, XP004275375 ISSN: 0167-4781
- DIEGO PRIETO ROCES ET AL: "EFFICACY OF ENZYME REPLACEMENT THERAPY IN ALPHA-MANNOSIDOSIS MICE: A PRECLINICAL ANIMAL STUDY", HUMAN MOLECULAR GENETICS, OXFORD UNIVERSITY PRESS, SURREY, vol. 13, no. 18, 15 September 2004 (2004-09-15), pages 1979-1988, XP008055443, ISSN: 0964-6906, DOI: 10.1093/HMG/DDH220

### SEQUENCE LISTING

<110> Jens Fogh
   Claes Anderson
   Cecilia Weigelt
   Diego Prieto Roces
   Kurt von Figura
   Meher Irani
<120> Use of alpha-mannosidase for the correction of abnormal lysosomal storage in the CNS
<130> 36564PC01
<150> PA200400528
   <151> 2004-04-01
<150> US 60/558,108
   <151> 2004-04-01
<160> 2
<170> FastSEQ for Windows Version 4.0
<210> 1
   <211> 1011
   <212> PRT
   <213> Homo Sapiens
<400> 1
<210> 2
   <211> 8079
   <212> DNA
   <213> Artificial Sequence
<220>
   <223> Expression plasmid pLamanExp1
<400> 2
   agatcttcaa tattggccat tagccatatt attcattggt tatatagcat aaatcaatat 60
   tggctattgg ccattgcata cgttgtatct atatcataat atgtacattt atattggctc 120
   atgtccaata tgaccgccat gttggcattg attattgact agttattaat agtaatcaat 180
   tacggggtca ttagttcata gcccatatat ggagttccgc gttacataac ttacggtaaa 240
   tggcccgcct ggctgaccgc ccaacgaccc ccgcccattg acgtcaataa tgacgtatgt 300
   tcccatagta acgccaatag ggactttcca ttgacgtcaa tgggtggagt atttacggta 360
   aactgcccac ttggcagtac atcaagtgta tcatatgcca agtccgcccc ctattgacgt 420
   caatgacggt aaatggcccg cctggcatta tgcccagtac atgaccttac gggactttcc 480
   tacttggcag tacatctacg tattagtcat cgctattacc atggtgatgc ggttttggca 540
   gtacaccaat gggcgtggat agcggtttga ctcacgggga tttccaagtc tccaccccat 600
   tgacgtcaat gggagtttgt tttggcacca aaatcaacgg gactttccaa aatgtcgtaa 660
   caactgcgat cgcccgcccc gttgacgcaa atgggcggta ggcgtgtacg gtgggaggtc 720
   tatataagca gagctcgttt agtgaaccgt cagatcacta gaagctttat tgcggtagtt 780
   tatcacagtt aaattgctaa cgcagtcagt gcttctgaca caacagtctc gaacttaagc 840
   tgcagtgact ctcttaaggt agccttgcag aagttggtcg tgaggcactg ggcaggtaag 900
   tatcaaggtt acaagacagg tttaaggaga ccaatagaaa ctgggcttgt cgagacagag 960
   aagactcttg cgtttctgat aggcacctat tggtcttact gacatccact ttgcctttct 1020
   ctccacaggt gtccactccc agttcaatta cagctcttaa ggctagagta cttaatacga 1080
   ctcactatag gctagcctcg agaattcgcc gccatgggcg cctacgcgcg ggcttcgggg 1140
   gtctgcgctc gaggctgcct ggactcagca ggcccctgga ccatgtcccg cgccctgcgg 1200
   ccaccgctcc cgcctctctg ctttttcctt ttgttgctgg cggctgccgg tgctcgggcc 1260
   gggggatacg agacatgccc cacagtgcag ccgaacatgc tgaacgtgca cctgctgcct 1320
   cacacacatg atgacgtggg ctggctcaaa accgtggacc agtactttta tggaatcaag 1380
   aatgacatcc agcacgccgg tgtgcagtac atcctggact cggtcatctc tgccttgctg 1440
   gcagatccca cccgtcgctt catttacgtg gagattgcct tcttctcccg ttggtggcac 1500
   cagcagacaa atgccacaca ggaagtcgtg cgagaccttg tgcgccaggg gcgcctggag 1560
   ttcgccaatg gtggctgggt gatgaacgat gaggcagcca cccactacgg tgccatcgtg 1620
   gaccagatga cacttgggct gcgctttctg gaggacacat ttggcaatga tgggcgaccc 1680
   cgtgtggcct ggcacattga ccccttcggc cactctcggg agcaggcctc gctgtttgcg 1740
   cagatgggct tcgacggctt cttctttggg cgccttgatt atcaagataa gtgggtacgg 1800
   atgcagaagc tggagatgga gcaggtgtgg cgggccagca ccagcctgaa gcccccgacc 1860
   gcggacctct tcactggtgt gcttcccaat ggttacaacc cgccaaggaa tctgtgctgg 1920
   gatgtgctgt gtgtcgatca gccgctggtg gaggaccctc gcagccccga gtacaacgcc 1980
   aaggagctgg tcgattactt cctaaatgtg gccactgccc agggccggta ttaccgcacc 2040
   aaccacactg tgatgaccat gggctcggac ttccaatatg agaatgccaa catgtggttc 2100
   aagaaccttg acaagctcat ccggctggta aatgcgcagc aggcaaaagg aagcagtgtc 2160
   catgttctct actccacccc cgcttgttac ctctgggagc tgaacaaggc caacctcacc 2220
   tggtcagtga aacatgacga cttcttccct tacgcggatg gcccccacca gttctggacc 2280
   ggttactttt ccagtcggcc ggccctcaaa cgctacgagc gcctcagcta caacttcctg 2340
   caggtgtgca accagctgga ggcgctggtg ggcctggcgg ccaacgtggg accctatggc 2400
   tccggagaca gtgcacccct caatgaggcg atggctgtgc tccagcatca cgacgccgtc 2460
   agcggcacct cccgccagca cgtggccaac gactacgcgc gccagcttgc ggcaggctgg 2520
   gggccttgcg aggttcttct gagcaacgcg ctggcgcggc tcagaggctt caaagatcac 2580
   ttcacctttt gccaacagct aaacatcagc atctgcccgc tcagccagac ggcggcgcgc 2640
   ttccaggtca tcgtttataa tcccctgggg cggaaggtga attggatggt acggctgccg 2700
   gtcagcgaag gcgttttcgt tgtgaaggac cccaatggca ggacagtgcc cagcgatgtg 2760
   gtaatatttc ccagctcaga cagccaggcg caccctccgg agctgctgtt ctcagcctca 2820
   ctgcccgccc tgggcttcag cacctattca gtagcccagg tgcctcgctg gaagccccag 2880
   gcccgcgcac cacagcccat ccccagaaga tcctggtccc ctgctttaac catcgaaaat 2940
   gagcacatcc gggcaacgtt tgatcctgac acagggctgt tgatggagat tatgaacatg 3000
   aatcagcaac tcctgctgcc tgttcgccag accttcttct ggtacaacgc cagtataggt 3060
   gacaacgaaa gtgaccaggc ctcaggtgcc tacatcttca gacccaacca acagaaaccg 3120
   ctgcctgtga gccgctgggc tcagatccac ctggtgaaga cacccttggt gcaggaggtg 3180
   caccagaact tctcagcttg gtgttcccag gtggttcgcc tgtacccagg acagcggcac 3240
   ctggagctag agtggtcggt ggggccgata cctgtgggcg acacctgggg gaaggaggtc 3300
   atcagccgtt ttgacacacc gctggagaca aagggacgct tctacacaga cagcaatggc 3360
   cgggagatcc tggagaggag gcgggattat cgacccacct ggaaactgaa ccagacggag 3420
   cccgtggcag gaaactacta tccagtcaac acccggattt acatcacgga tggaaacatg 3480
   cagctgactg tgctgactga ccgctcccag gggggcagca gcctgagaga tggctcgctg 3540
   gagctcatgg tgcaccgaag gctgctgaag gacgatggac gcggagtatc ggagccacta 3600
   atggagaacg ggtcgggggc gtgggtgcga gggcgccacc tggtgctgct ggacacagcc 3660
   caggctgcag ccgccggaca ccggctcctg gcggagcagg aggtcctggc ccctcaggtg 3720
   gtgctggccc cgggtggcgg cgccgcctac aatctcgggg ctcctccgcg cacgcagttc 3780
   tcagggctgc gcagggacct gccgccctcg gtgcacctgc tcacgctggc cagctggggc 3840
   cccgaaatgg tgctgctgcg cttggagcac cagtttgccg taggagagga ttccggacgt 3900
   aacctgagcg cccccgttac cttgaacttg agggacctgt tctccacctt caccatcacc 3960
   cgcctgcagg agaccacgct ggtggccaac cagctccgcg aggcagcctc caggctcaag 4020
   tggacaacaa acacaggccc cacaccccac caaactccgt accagctgga cccggccaac 4080
   atcacgctgg aacccatgga aatccgcact ttcctggcct cagttcaatg gaaggaggtg 4140
   gatggttagg tctgctggga tgggccctct agagtcgacc cgggcggccg cttcccttta 4200
   gtgagggtta atgcttcgag cagacatgat aagatacatt gatgagtttg gacaaaccac 4260
   aactagaatg cagtgaaaaa aatgctttat ttgtgaaatt tgtgatgcta ttgctttatt 4320
   tgtaaccatt ataagctgca ataaacaagt taacaacaac aattgcattc attttatgtt 4380
   tcaggttcag ggggagatgt gggaggtttt ttaaagcaag taaaacctct acaaatgtgg 4440
   taaaatccga taaggatcga tccgggctgg cgtaatagcg aagaggcccg caccgatcgc 4500
   ccttcccaac agttgcgcag cctgaatggc gaatggacgc gccctgtagc ggcgcattaa 4560
   gcgcggcggg tgtggtggtt acgcgcagcg tgaccgctac acttgccagc gccctagcgc 4620
   ccgctccttt cgctttcttc ccttcctttc tcgccacgtt cgccggcttt ccccgtcaag 4680
   ctctaaatcg ggggctccct ttagggttcc gatttagagc tttacggcac ctcgaccgca 4740
   aaaaacttga tttgggtgat ggttcacgta gtgggccatc gccctgatag acggtttttc 4800
   gccctttgac gttggagtcc acgttcttta atagtggact cttgttccaa actggaacaa 4860
   cactcaaccc tatctcggtc tattcttttg atttataagg gattttgggg atttcggcct 4920
   attggttaaa aaatgagctg atttaacaaa aatttaacgc gaattaattc tgtggaatgt 4980
   gtgtcagtta gggtgtggaa agtccccagg ctccccaggc aggcagaagt atgcaaagca 5040
   tgcatctcaa ttagtcagca accaggtgtg gaaagtcccc aggctcccca gcaggcagaa 5100
   gtatgcaaag catgcatctc aattagtcag caaccatagt cccgccccta actccgccca 5160
   tcccgcccct aactccgccc agttccgccc attctccgcc ccatggctga ctaatttttt 5220
   ttatttatgc agaggccgag gccgcctctg cctctgagct attccagaag tagtgaggag 5280
   gcttttttgg aggcctaggc ttttgcaaaa agctcccggg atggttcgac cattgaactg 5340
   catcgtcgcc gtgtcccaaa atatggggat tggcaagaac ggagacctac cctggcctcc 5400
   gctcaggaac gagttcaagt acttccaaag aatgaccaca acctcttcag tggaaggtaa 5460
   acagaatctg gtgattatgg gtaggaaaac ctggttctcc attcctgaga agaatcgacc 5520
   tttaaaggac agaattaata tagttctcag tagagaactc aaagaaccac cacgaggagc 5580
   tcattttctt gccaaaagtt tggatgatgc cttaagactt attgaacaac cggaattggc 5640
   aagtaaagta gacatggttt ggatagtcgg aggcagttct gtttaccagg aagccatgaa 5700
   tcaaccaggc caccttagac tctttgtgac aaggatcatg caggaatttg aaagtgacac 5760
   gtttttccca gaaattgatt tggggaaata taaacttctc ccagaatacc caggcgtcct 5820
   ctctgaggtc caggaggaaa aaggcatcaa gtataagttt gaagtctacg agaagaaaga 5880
   ctaattcgaa atgaccgacc aagcgacgcc caacctgcca tcacgatggc cgcaataaaa 5940
   tatctttatt ttcattacat ctgtgtgttg gttttttgtg tgaatcgata gcgataagga 6000
   tccgcgtatg gtgcactctc agtacaatct gctctgatgc cgcatagtta agccagcccc 6060
   gacacccgcc aacacccgct gacgcgccct gacgggcttg tctgctcccg gcatccgctt 6120
   acagacaagc tgtgaccgtc tccgggagct gcatgtgtca gaggttttca ccgtcatcac 6180
   cgaaacgcgc gagacgaaag ggcctcgtga tacgcctatt tttataggtt aatgtcatga 6240
   taataatggt ttcttagacg tcaggtggca cttttcgggg aaatgtgcgc ggaaccccta 6300
   tttgtttatt tttctaaata cattcaaata tgtatccgct catgagacaa taaccctgat 6360
   aaatgcttca ataatattga aaaaggaaga gtatgagtat tcaacatttc cgtgtcgccc 6420
   ttattccctt ttttgcggca ttttgccttc ctgtttttgc tcacccagaa acgctggtga 6480
   aagtaaaaga tgctgaagat cagttgggtg cacgagtggg ttacatcgaa ctggatctca 6540
   acagcggtaa gatccttgag agttttcgcc ccgaagaacg ttttccaatg atgagcactt 6600
   ttaaagttct gctatgtggc gcggtattat cccgtattga cgccgggcaa gagcaactcg 6660
   gtcgccgcat acactattct cagaatgact tggttgagta ctcaccagtc acagaaaagc 6720
   atcttacgga tggcatgaca gtaagagaat tatgcagtgc tgccataacc atgagtgata 6780
   acactgcggc caacttactt ctgacaacga tcggaggacc gaaggagcta accgcttttt 6840
   tgcacaacat gggggatcat gtaactcgcc ttgatcgttg ggaaccggag ctgaatgaag 6900
   ccataccaaa cgacgagcgt gacaccacga tgcctgtagc aatggcaaca acgttgcgca 6960
   aactattaac tggcgaacta cttactctag cttcccggca acaattaata gactggatgg 7020
   aggcggataa agttgcagga ccacttctgc gctcggccct tccggctggc tggtttattg 7080
   ctgataaatc tggagccggt gagcgtgggt ctcgcggtat cattgcagca ctggggccag 7140
   atggtaagcc ctcccgtatc gtagttatct acacgacggg gagtcaggca actatggatg 7200
   aacgaaatag acagatcgct gagataggtg cctcactgat taagcattgg taactgtcag 7260
   accaagttta ctcatatata ctttagattg atttaaaact tcatttttaa tttaaaagga 7320
   tctaggtgaa gatccttttt gataatctca tgaccaaaat cccttaacgt gagttttcgt 7380
   tccactgagc gtcagacccc gtagaaaaga tcaaaggatc ttcttgagat cctttttttc 7440
   tgcgcgtaat ctgctgcttg caaacaaaaa aaccaccgct accagcggtg gtttgtttgc 7500
   cggatcaaga gctaccaact ctttttccga aggtaactgg cttcagcaga gcgcagatac 7560
   caaatactgt ccttctagtg tagccgtagt taggccacca cttcaagaac tctgtagcac 7620
   cgcctacata cctcgctctg ctaatcctgt taccagtggc tgctgccagt ggcgataagt 7680
   cgtgtcttac cgggttggac tcaagacgat agttaccgga taaggcgcag cggtcgggct 7740
   gaacgggggg ttcgtgcaca cagcccagct tggagcgaac gacctacacc gaactgagat 7800
   acctacagcg tgagctatga gaaagcgcca cgcttcccga agggagaaag gcggacaggt 7860
   atccggtaag cggcagggtc ggaacaggag agcgcacgag ggagcttcca gggggaaacg 7920
   cctggtatct ttatagtcct gtcgggtttc gccacctctg acttgagcgt cgatttttgt 7980
   gatgctcgtc aggggggcgg agcctatgga aaaacgccag caacgcggcc tttttacggt 8040
   tcctggcctt ttgctggcct tttgctcaca tggctcgac                        8079

## Description

### FIELD OF INVENTION

The present invention provides means and strategies for treating the lysosomal storage disorder alpha-mannosidosis by enzyme replacement therapy. In particular, the invention pertains to the reduction of stored neutral mannose-rich oligosaccharides in cells within the central nervous system.

### BACKGROUND OF THE INVENTION

### Alpha-mannosidosis

Alpha-mannosidosis is a recessive, autosomal disease that occurs world wide with a frequency of between 1/1.000.000 and 1/500.000. Mannosidosis is found in all ethnic groups in Europe, America, Africa and also Asia. It is detected in all countries with a good diagnostic service for lysosomal storage disorders, at a similar frequency. They are born apparently healthy, however the symptoms of the diseases are progressive. Alpha-mannosidosis displays clinical heterogeneity, ranging from very serious to very mild forms. Typical clinical symptoms are: mental retardation, skeletal changes, impaired immune system resulting in recurrent infections, hearing impairment and often the disease is associated with a typical facial characteristics such as a coarse face, a prominent forehead, a flattened nasal bridge, a small nose, and a broad mouth. In the most severe cases (mannosidosis type I) the children suffer from hepatosplenomegaly, and they die during the first years of life. Possibly this early death is caused by severe infections due to the immunodeficiency caused by the disease. In milder cases (mannosidosis type 2) the patients usually reach adult age. The skeletal weaknesses of the patients result in the needs of wheeling chairs at age 20 to 40. The disease causes a diffuse dysfunction of the brain often resulting in weak mental performances that excludes anything but the most basic skills of simple reading and writing. These problems associated with hearing inabilities and other clinical manifestations preclude the patient from an independent life, the consequence being that life long caretaking is needed.

### Lysosomal alpha-mannosidase

Alpha-mannosidosis results from a deficient activity of lysosomal alpha-mannosidase (LAMAN, EC3.2.1.24). The disease is characterised by massive intracellular accumulation of mannose-rich oligosaccharides, that is oligosaccharides carrying α1,2-, α1,3- and α1,6-mannosyl residues at their non-reducing termini. These oligosaccharides mainly originate from the intralysosomal degradation of glycoproteins with N-linked oligosaccharides. However, some originate from the catabolism of dolichol-linked oligosaccharides and from misfolded glycoproteins redirected to the cytosol for degradation by the proteasome (4, 5). The lysosomal storage is observed in a wide range of cell types and tissues, including neurons in all brain regions. LAMAN is an exoglycosidase which hydrolyses these terminal, non-reducing alpha-D-mannose residues in alpha-D-mannosides from the non-reducing end during the ordered degradation of the N-linked glycoproteins (Aronson and Kuranda FASEB J 3:2615-2622. 1989). The human enzyme is synthesised as a single polypeptide of 1011 amino acids with a putative signal peptide of 49 residues that is processed into three main glycopeptides of 15, 42, and 70 kD. (Nilssen et al. Hum.Mol.Genet. 6, 717-726. 1997).

### The lysosomal alpha-mannosidase gene

The gene coding for LAMAN (MANB) is located at chromosome 19 (19cen-q12), (Kaneda et al. Chromosoma 95:8-12. 1987). MANB consists of 24 exons, spanning 21.5 kb (GenBank accession numbers U60885-U60899; Riise et al. Genomics 42:200-207 . 1997). The LAMAN transcript is » 3,500 nucleotides (nts) and contains an open reading frame encoding 1,011 amino acids (GenBank U60266.1).

The cloning and sequencing of the human cDNA encoding LAMAN has been published in three papers (Nilssen et al. Hum.Mol.Genet. 6, 717-726. 1997; Liao et al. J.Biol.Chem. 271, 28348-28358. 1996; Nebes et al. Biochem.Biophys.Res.Commun. 200, 239-245. 1994). Curiously, the three sequences are not identical. When compared to the sequence of Nilssen et al (accession # U60266.1) a TA to AT change at positions 1670 and 1671 resulting in a valine to asparitic acid substitution was found by Liao et al. and Nebes et al.

### Mutations

In 1997 Nilssen et al. (Hum Mol Genet 6:717-726) reported the first disease causing mutation in the fibroblasts from two siblings with mannosidosis in a highly inbred Palestinian family. In these two patients, the cause of the disease is a point mutation at the gene, with the nucleotide Adenine substituted with Thymidin. Since then, several genetic causes to the disease have been found in man. Today mutations causing alpha-mannosidosis have been characterised in more than 60 patients, manly in Europeans. These mutations vary both in type, encompassing nucleotide substitutions, deletions and additions and spanning most of the 24 exons of the gene (Berg et al. Am J Genet 64: 77-88. 1999).

The consequences of the mutations on the protein level also vary, from amino acid substitutions that effect both the active site and the folding, to early frame shifts and premature stop codons, resulting in a completely inactive product.

The amino acid sequence and enzyme structure in a number of different species. When comparing species, many of the human and the bovine mutations found are located to a highly "conserved" region of the mannosidase polypeptide for several species like man, cattle, cat, whale, bird, cod and slime mould, indicating that these regions are very important for the enzyme function.

Despite the many different types of mutations, it seems that all the mutations cause the similar complete loss of enzyme activity in cells. Thus, apparently, the variations with respect to clinical severity cannot be explained by variations in the enzyme activity. Rather, these variations are caused by other factors, being attributed to the general ability of the organism to buffer the obnoxious effects of the accumulating oligomannosides, and by the environments to prevent and to treat infections and other clinical symptoms in the patients.

### Diagnosis

The diagnosis of alpha-mannosidosis is currently is based on clinical evaluation, detection of mannose-rich oligosaccharides in urine, and direct measurements of alpha-mannosidase activity in various cell types, such as leukocytes, fibroblasts, and amniocytes (Chester et al., In: Durand P, O'Brian J (eds) Genetic errors of glycoprotein metabolism. Edi-Ermes, Milan, pp 89-120. 1982; Thomas and Beaudet . In: Scriver CR, Beaudet AL, Sly WA, Valle D (eds) The metabolic and molecular bases of inherited disease. Vol 5. McGraw-Hill, New York, pp 2529-2562. 1995).

Because the symptoms initially often are mild and the biochemical diagnosis is difficult, the diagnosis is frequently made late in the course of the disease. It is obvious that patients and their families would benefit substantially from an early diagnosis.

### Animal models

Alpha mannosidosis has been described in cattle (Hocking et al. Biochem J 128:69-78. 1972), cats (Walkley et al. Proc. Nat. Acad. Sci. 91: 2970-2974, 1994), and guinea pigs (Crawley et al. Pediatr Res 46: 501-509, 1999). A mouse model was recently generated by targeted disruption of the alpha-mannosidase gene (Stinchi et al. Hum Mol Genet 8: 1366-72, 1999).

Like in humans alpha mannosidase seems to be caused by specific mutations in the gene coding for lysosomal alpha-mannosidase. Berg et al. (Biochem J. 328:863-870.1997) reported the purification of feline liver lysosomal alpha-mannosidase and determination of its cDNA sequence. The active enzyme consists of 3 polypeptides, with molecular masses of 72, 41, and 12 kD. Similary to the human enzyme it was demonstrated that the feline enzyme is synthesized as a single-chain precursor with a putative signal peptide of 50 amino acids followed by a polypeptide chain of 957 amino acids, which is cleaved into the 3 polypeptides of the mature enzyme. The deduced amino acid sequence was 81.1% and 83.2% identical with the human and bovine sequences, respectively. A 4-bp deletion was identified in an affected Persian cat; the deletion resulted in a frameshift from codon 583 and premature termination at codon 645. No enzyme activity could be detected in the liver of the cat. A domestic long-haired cat expressing a milder phenotype had enzyme activity of 2% of normal; this cat did not possess the 4-bp deletion. Tollersrud et al. (Eur J Biochem 246:410-419 .1997) purified the bovine kidney enzyme to homogeneity and cloned the gene. The gene was organized in 24 exons that spanned 16 kb. Based on the gene sequence they identified two mutations in cattle.

At this time the α-mannosidase knock-out mice provide the best available pharmacological model for investigative treatment of α-mannosidosis to support regulatory submission. Acceptable models, which do not use live animals currently, do not exist.

### Medical need for alpha-mannosidosis therapy

In light of the severe clinical manifestations resulting form the accumulation of mannose-rich oligosaccharides, the lack of effective treatment for alpha-mannosidosis is well recognised. At present, the major therapeutic options for treatment of the disease are bone marrow transplantation and enzyme replacement therapy.

### Bone marrow transplantation

In 1996 Walkley et al. (Proc. Nat. Acad. Sci. 91: 2970-2974, 1994) published a paper on 3 kittens with mannosisdosis that were treated with bone marrow transplantation (BMT) in 1991. In the 2 animals that were sacrificed a normalisation was seen, not only in the body, but more importantly, also in brain. The 3'rd cat was well after 6 years. Normally, an untreated cat dies with 3-6 months. In 1987 a child with mannosidosis was treated with BMT (Will et al. Arch Dis Child 1987 Oct;62(10):1044-9). He died after 18 weeks due to procedure related complications. In brain little enzyme activity was found. This disappointing result could be explained by heavy immunosuppressive treatment before death, or that it takes time for the enzyme activity to increase in brain after BMT. The donor was the mother (who as carrier must be expected to have less than 50% enzyme activity) or it may be BMT in man has no effect on enzyme function in brain. Despite having variable outcomes the few attempts of bone marrow transplantation have thus indicated that successful engraftment can correct the clinical manifestations of alpha mannosidosis, at least in part. However, the challenge of reducing the serious procedure related complications when applying bone marrow transplantation i human therapy still remains to be defeated.

### Enzyme replacement therapy

When lysosomal storage diseases were discovered, hopes were raised that this could be treated by enzyme substitution. Enzyme replacement therapy has proven efficient in Gaucher disease. When exogenous lysosomal glucocerebrosidase is injected into the patient, this enzyme is taken up by enzyme-deficient cells (Barton et al. N Engl J Med 324:1464-1470). Such uptake is regulated by certain receptors on the cell surface as for instance the mannose-6-phosphate receptor, which is nearly ubiquitous on the surface of cells and other receptors such as the asialoglycoprotein receptor and the mannose receptor, which are restricted to certain cell types such as cells of the monocyte/macrophage cell line and hepatocytes. The cellular uptake of the enzyme is therefore heavily dependent upon its glycosylation profile. If properly designed, the deficient enzyme could be replaced by regular injections of exogenous enzyme in the same manner as diabetic patients receive insulin. *In vitro* studies with the purified active lysosomal alpha-mannosidase added to the media of enzyme-deficient fibroblasts showed correction of the lysosomal substrate accumulation. *In vivo* treatment, on the other hand, has been hampered in part by the problem of producing the sufficient quantity of enzymes, and by complications resulting from immune reactions against the exogenous enzyme. Most importantly, however, special considerations apply in relation to lysosomal storage diseases with a major neurological component, such as alpha-mannosidosis, wherein the clinical manifestations are related to increased lysosomal storage within the central nervous system. Thus, enzyme replacement therapy has not proven effective against the acute neuronopathic variant of Gaucher disease (Prows et al. Am J Med Genet 71:16-21).

The delivery of therapeutic enzymes to the brain is prevented by the absence of transport of these large molecules through the blood-brain barrier. From the general notion that the blood brain barrier must be circumvented in order to see an effect of therapeutic agents in the brain, the use of a large diversity of delivery systems have been contemplated. These include invasive techniques such as osmotic opening of the blood brain barrier with for instance mannitol and non-invasive techniques such as receptor mediated endocytosis of chimeric enzymes. As enzyme replacement is expected to require administration of the enzyme on a regular basis, the use of invasive techniques should be avoided. Use of the non-invasive techniques, on the other hand, has not provided promising results. It has been contemplated that reduced storage in visceral organs and in the meninges could reduce the amount of oligosaccharides that is carried to the brain. Such considerations, however, are not considered to be applicable to lysosomal disorders in which the neurological damage is primary and severe (Neufeld, E. F. Enzyme replacement therapy, in "Lysosomal disorders of the brain (Platt, F. M. Walkley, S. V: eds Oxfor University Press). Accordingly, the quest for an effective treatment of alpha -mannosidosis is still ongoing.

WO 02/099092 (Fogh et al., 2002) and Berg et al., Molecular Genetics and Metabolism, vol. 73, no. 1, April 2001, pages 18-29, discloses the use of a lysosomal alpha-mannosidase (LAMAN) for the preparation of a medicament for reducing the intracellular levels of mannose-rich oligosaccharides within the CNS by administering LAMAN "over cellular membranes".

Fogh *et a*/*.,* 2002, discusses enzyme replacement therapy and discloses that if properly designed the missing enzyme could be injected regularly like the diabetic subject injects insulin. Moreover, Fogh *et al.,* 2002 and Berg *et al.,* 2001, disclose expression and purification of human LAMAN from CHO cells and demonstrate in vitro the uptake of LAMAN in fibroblasts of a patient with mannosidosis. However, Fogh *et al.,* 2002, and Berg *et al.,* 2001, do not disclose administration by repeated intravenous injection at a specific frequency.

### SUMMARY OF THE INVENTION

A first aspect of the present invention provides the use of a lysosomal alpha-mannosidase for the preparation of a medicament for treatment of alpha-mannosidosis, wherein said medicament is to be administered by intravenous administration.

An embodiment of the present invention pertains to the use, wherein the medicament is to be administered by
infusion, or
repeated intravenous injection in a frequency corresponding to 1 to 5 daily injections, 1 to 5 injections per week, 1 to 5 injections every 2 weeks or 1 to 5 injections every month.

A second embodiment of the present invention pertains to the use, wherein said medicament does not comprise a component with a known ability to target cells within the central nervous system.

A third embodiment of the present invention pertains to the use, wherein the lysosomal alpha-mannosidase comprises the amino acid sequence of SEQ ID NO: 1.

A further embodiment of the present invention pertains to the use, wherein said medicament does not comprise a component for targeting of cells within the central nervous system and does not comprise a component with the ability to act as vehicle for the passage over the blood-brain barrier.

Another embodiment of the present invention pertains to the use, wherein said medicament is administered in an amount, which is within the range of 5 to 300 mU lysosomal alpha-mannosidase per gram body weight of the subject to be treated.

Yet another embodiment of the present invention pertains to the use, wherein a fraction of said lysosomal alpha-mannosidase consists of lysosomal alpha-mannosidase having one or more N-linked oligosaccharides carrying mannose 6-phosphate residues.

A preferred embodiment of the present invention pertains to the use, wherein said lysosomal alpha-mannosidase is a recombinant enzyme.

### DEFINITIONS AND TERMS

By "component" is meant, in broad terms, an element forming a part of the whole. More specifically, the term refers to a substance present in a mixture. Such a substance may be a compound defined as a material formed from elements chemically combined in definite proportions by mass. Examples of compounds inorganic compounds such as small molecular species, organic compounds such as carbohydrates including simple sugars and complex polysaccharide polymers, peptides, proteins, peptidomimetics, nucleic acids and antibodies.

The term "central nervous system" refers to the brain, spinal cord, and spinal nerves. The central nervous system does not include the peripheral nerves in the arms, legs, muscles, and organs.

In the present context the term "brain" refers to that portion of the central nervous system that is located within the skull including its two (right and left) halves referred to as the hemispheres.

Also in the present context, extraneural cells such as cells in endothelia and epithelia of the choroid plexus are not to be considered parts of the central nervous system and the brain. Also the brain and central nervous system do not comprise the meninges.

The "choroid plexus" is a vascular proliferation or fringe of the tela choroidea in the third, fourth, and lateral cerebral ventricles; it secretes cerebrospinal fluid, thereby regulating to some degree the intraventricular pressure.

The term "meninges" refer to the three membranes that cover the brain and spinal cord. The outside meninx is called the dura mater, and is the most resilient of the three. The center layer is the pia mater, and the thin innermost layer is the arachnoid.

By "complex" or "conjugate" is meant a molecule or a group of molecules which are either held together by van der Waal's and/or electrostatic and/or by covalent bonds.

The term "exogenous" refers to a component which is introduced from or produced outside the organism or system; specifically, the term refers to components not synthesized within the organism or system.

The term "permeant" refers to a component, which is able to pass through a particular semipermeable membrane. Such a semipermeable membranes may be the blood-brain barrier and/or the foetal-maternal barrier.

The terms "blood-brain barrier" and "blood-cerebral barrier" refer to the barrier system separating the blood from the parenchyma of the central nervous system.

The term "targeting" refers to the process of process of having proteins contain certain signals such that the proteins are directed specifically towards certain cellular locations, e.g., the lysosome.

### DETAILED DESCRIPTION

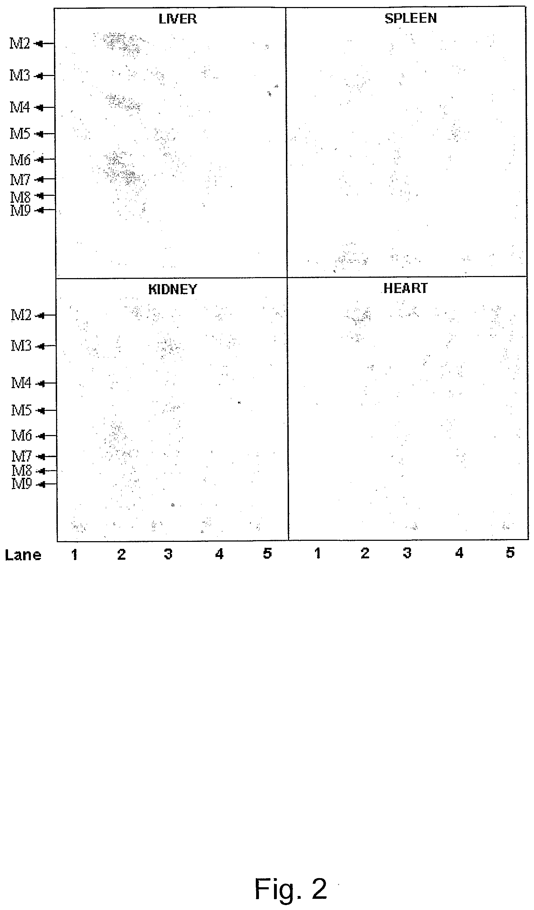

The hallmark of α-mannosidosis is the storage of neutral oligosaccharides in a wide variety of tissues (see Fig. 2, lane 1 and lane 2). These oligosaccharides contain 2-9 mannose residues and an N-acetylglucosamine residue at their reducing terminus and therefore result from the action of an endoglucosaminidase. The heavy involvement of the central nervous system is reflected in the clinical phenotype of alpha-mannosidosis ranging from severe infantile forms to milder juvenile forms with only moderate mental retardation. The rationale behind the present invention emerges from the surprising observation that repeated intravenous administration of recombinant alpha-mannosidase results in a marked reduction in the levels of stored neutral mannose-rich oligosaccharides in the brain.

In its broadest aspect, the present disclosure relates to a recombinant LAMAN polypeptide and to a nucleotide sequence encoding the LAMAN polypeptide, to an expression system capable of expressing the polypeptide as well as to pharmaceutical compositions comprising the polypeptide or part of it and to the use of the polypeptide for preventing or treating the development of symptoms related to alpha-mannosidosis caused by a deficiency, in a subject, of the Lysosomal alpha-mannosidase (LAMAN) enzyme.

In one main aspect, the invention provides the use of a lysosomal alpha-mannosidase for the preparation of a medicament for reducing the intracellular levels of neutral mannose-rich oligosaccharides in cells within one or more regions of the central nervous system, such as one or more regions within the brain. The neutral mannose-rich oligosaccharides contain two to nine mannose residues linked to a single N-acetylglucosamine residue.

Another main aspect of the invention pertains to a formulation of a lysosomal alpha-mannosidase for use as a medicament for reducing the intracellular levels of neutral mannose-rich oligosaccharides in cells within one or more regions of the central nervous system.

For both main aspects of the invention it is further preferred that the lysosomal alpha-mannosidase is selected from the group consisting of
(a) the amino acid sequence of SEQ ID NO:1;
(b) a portion of the sequence in (a), which is enzymatically equivalent to recombinant human lysosomal alpha-mannosidase
(c) an amino acid sequence analogue having at least 75% sequence identity to any one of the sequences in (a) or (b) and at the same time being enzymatically equivalent to recombinant human lysosomal alpha-mannosidase.

It may be preferred that the degree of sequence identity between the nucleic acid sequence comprised within the cell according to the invention and SEQ ID NO: 1 is at least 80%, such as at least 85%, at least 90%, at least 95%, at least 97%, at least 98%, or at least 99%.

In the present context, an amino acid sequence analogue or a portion of an amino acid sequence, which is enzymatically equivalent to recombinant human lysosomal alpha-mannosidase, is a polypeptide, which has a specific activity of at least 5 U/mg when present at a purity of at least 90%. Purity is conveniently determined using conventional techniques such as SDS-PAGE. Specific activity may be determined in an enzyme assay as described in example 2 of the present application. In brief, the purified polypeptide is incubated with the substrate, p-nitrophenyl-α-mannopyranoside at 37°C for an appropriate amount of time before the reaction is stopped by the addition of 0.4 M glycine/NaOH pH 10.4. The amount of substrate converted may be determined by the change of absorbance at 405 nm (ε=18500 M⁻¹ cm⁻¹).

In some embodiments the lysosomal alpha-mannosidase is formulated for systemic delivery. Formulations for systemic delivery may comprise a variety of pharmaceutically acceptable carriers, excipients or stabilizers (Remington's Pharmaceutical Sciences 16th edition, Osol, A. Ed. (1980)), in the form of lyophilized formulations or aqueous solutions. "Pharmaceutically acceptable" carriers, excipients, or stabilizers are nontoxic to recipients at the dosages and concentrations employed, and for the present applications they are also present in concentrations which have no effect on the permeability of the blood-brain barrier. Included are buffers such as phosphate, citrate, and other organic acids; antioxidants including ascorbic acid and methionine; preservatives (such as octadecyidimethylbenzyl ammonium chloride; hexamethonium chloride; benzalkonium chloride, benzethonium chloride: phenol, butyl or benzyl alcohol; alkyl parabens such as methyl or propyl paraben; catechol; resorcinol; cyclohexanol; 3-pentanol; and m-cresol); low molecularweight (less than about 10 residues) polypeptide; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e. g., Zn-protein complexes); and/or non-ionic surfactants such as TWEEN, PLURONICST or polyethylene glycol (PEG).

Successful therapy of alpha-mannosidosis must effectively address the challenges posed by the central nervous system and the blood-brain barrier in particular as it prevents the entry of lysosomal enzymes into the brain. The anatomical component of the blood brain barrier consists of unique endothelial cells in the brain capillaries, having tight junctions without fenestrations and with few microvilli and few vesicles for fluid transport. Its physiologic component in part consists of enzymes unique to the brain endothelia and of active transport via carrier proteins.

Beyond the blood-brain barrier additional barriers are present. Perivascular spaces are patrolled by phagocytes that can sequester foreign compounds, and furthermore, layers of basal laminae, macromolecular meshes of protein lie between the vasculature and central nervous system parenchyma. After this, the *glia lamintan,* a sheath made up of the end feet of astroglia and microglia forms another physical barrier. Finally, solutes beyond the endothelial barrier are also subject to an outward bulk fluid flow through contiguous spaces. Interstitial fluid from the central nervous system parenchyma moves partly into the ventricular system and perivascular spaces and subsequently solutes are carried to the subarachnoid spaces from which soluble constituents return to the bloodstream. Thus multiple barriers exist that limit and regulate the entry into of elements from the blood stream into the brain.

It has been contemplated that the blood brain barrier problem may be solved with the development of brain drug targeting technology. This technology relies on the delivery of therapeutic proteins to the brain via access on endogenous transport systems localized within the brain capillary endothelium, which forms the blood-brain barrier in vivo.

In the following non-limiting examples of components with an ability to target or mediate targeting of the central nervous system and/or with a known ability to act as vehicles for the passage over the blood-brain barrier delivery and/or cellular membranes are given.
1) Peptides and proteins as vehicles for passage LAMAN to the target cells by passage over cell membranes and/or the BBB:
   A number of studies in animals have shown that certain proteins and/or peptides may act as vehicles for passage of BBB. For instance proteins modified by the insulin fragment (Fukuta et al. Phaomacol Res 11: 1681-1688) or antibodies to the transferrin receptor (Friden et al. Proc Natl Acad Sci USA 88: 4771-4775) can pass the blood-brain barrier. Also proteins modified by coupling to polyamines (Poduslo and Curran. J Neurochem 66: 1599-1606) have been reported to pass the blood-brain barrier. In addition, Peptidomimetic monoclonal antibodies (MAb) undergo receptor-mediated transcytosis (RMT) through the BBB in vivo via transport on the endogenous BBB transferrin receptor (TfR) or insulin receptor (IR). Therapeutic proteins may be conjugated to the MAb transport vectors with either avidin-biotin technology or as fusion proteins.
2) Toxins as vehicles for passage LAMAN to the target cells by passage over cell membranes and/or the BBB:
   Different bacteria, plants and animals produce toxins. Toxins have many different targets such as the gut (enterotoxins), nerves or synapses (neurotoxins). Toxins can traverse cell membranes via receptor mediated processes and the embodiment of the present invention is to use toxins as vehicles to passage rhLAMAN to the target cells over cellular membranes and/or the BBB. The preferred target cells are cells in the CNS and/or the peripheral nervous system. It is to be understood that only the part of the toxin that is responsible for association with and translocation over cellular membranes is used.
      One example of a toxin used as a vehicle is a bacterial toxin such as Diphtheria Toxin (DT), from the *Corynebacterium Diptheriae.* Bacterial toxins exhibit a wide range of toxicities and they fall into groups by structure and function. The toxin binds to a target cell and enters the cell via a receptor, and is reduced to separate fragments. The processed toxin can be divided into the following 3 domains: The catalytic domain (C), the receptor domain (R), and the translocation domain (T).
      The catalytic fragment and the receptor fragment of the toxin or fragments thereof are replaced by the LAMAN. This fusion protein can traverse cellular membranes and/or the BBB and thereby deliver the LAMAN to the target cells. One example of the engineering of a hybrid molecule could be by recombinant technology
      Other examples of bacterial toxins to be used as vehicles are Clostridium Botulinum, Pseudomonas Exotoxin A produced by *Psedomonas aeruginosa,* Cholera Toxin produced by *Vibrio cholerae,* and Pertussis Toxin produced by *Bordetella pertussis.*
      Further examples of toxins used as vehicles are plant toxins selected from the list of the following plant toxins: cholinesterase inhibitors, protease inhibitors, amylase inhibitors, tannins, cyanogenic glycosides, goitrogens, lectin proteins, and lathyrogens, pyrrozidine alkaloids.
      Yet further examples of toxins used as vehicle are toxins from shellfish (saxitoxin) and snakes (alpha-bungarotoxin). The skilled person may add further examples in light of the details and characteristics of the invention.
3) Proteins and/or peptides isolated from bacteria or viruses as vehicles for passage rhLAMAN to the target cells by passage over cell membranes and/or the BBB:
   The transacting element and/or transacting protein from bacteria or viruses can be used together with rhLAMAN to cross the BBB and/or cellular membranes.
      Examples of bacteria are selected from the following list: Ns. meningitidis, S. pneumoniae, Hemophilus influenzae, Staphylococcus species, Proteus species, Pseudomonas species, E. coli, Listeria monocytogenes, M. Tuberculosis, Neurolues, Spirochetes Borrelia burgdorferi from Iodex ricinus.
      Examples of viruses are selected from the following list of virus families: Parvoviridae, Papovaviridae, Adenoviridae, Herpesviridae, Poxviridae, Picornaviridae, Reoviridae, Togaviridae, Arenaviridae, Coronaviridae, Retroviridae, Bunyaviridae, Orthomyxoviridae, Paramyxoviridae, and Rhabdoviridae. Relevante examples of viruses selected from the list mentioned above are e.g. Measles virus, Papova virus, and JC virus.
4) Cell mediated delivery systems
   A cell-mediated approach to therapy may address storage disorders in more than one fundamental way. The first is to replace or compensate for defective cell populations with normal equivalents. A second way to obtain therapeutic benefit can be through cross correction. Appropriate cell types offer the ability to not only cross the endothelial barrier from the blood but to actively migrate into the parenchyma and take up residence within the milieu of the diseased cells. The preferred human cell is selected from human monocytes, human fibroblasts, and human lymphocytes.
5) Disturbing the Blood-Brain Barrier
   Another invasive strategy for enhanced CNS drug delivery involves the systemic administration of drugs in conjunction with transient BBB disruption (BBBD). Theoretically, with the BBB weakened, systemically administered drugs can undergo enhanced extravasation rates in the cerebral endothelium, leading to increased parenchymal drug concentrations. A variety of techniques that transiently disrupt the BBB have been investigated; however, albeit physiologically interesting, many are unacceptably toxic and therefore not clinically useful. These include the infusion of solvents such as dimethyl sulfoxide or ethanol and metals such as aluminium; X-irradiation; and the induction of pathological conditions including hypertension, hypercapnia, hypoxia or ischemia.

A somewhat safer technique involves the systemic delivery of the convulsant drug, metrazol, which transiently increases the BBB permeability while causing seizures. Concurrent administration of the anticonvulsant pentobarbital blocks seizing while allowing BBBD to persist. The BBB can also be compromised by the systemic administration of several antineoplastic agents including VP-16, cisplatin, hydroxylurea, flurouracil and etoposide.

### Osmotic Blood-Brain Barrier Disruption

Intracarotid injection of an inert hypertonic solution such as mannitol or arabinose has been employed to initiate endothelial cell shrinkage and opening of BBB tight junctions for a period of a few hours, and this permits delivery of antineoplastic agents to the brain. Osmatic distruption has been tested as a strategy for the delivery of macromolecular drugs such as monocolonal antibodies, nanoparticles and viruses. However, the procedure breaks down the self-defense mechanism of the brain and levels it vulnerable to damage or infection from all circulating chemicals or toxins. The risk factors include, the passage of plasma proteins, the altered glucose uptake, the expression of heat shock proteins, microembolism or abnormal neuronal function. While a large number of techniques have thus been developed for facilitating the delivery of compounds over the blood brain barrier many of them show limited success in addressing the other barriers mentioned above. Their applicability in the treatment of alpha-mannosidosis therefore appears limited.

In contrast, a very important aspect of the invention however pertains to a scheme for the reduction of the intralysosomal accumulation of neutral mannose-rich oligosasccharides within the central nervous system based on a technique, which is not dependent on means for mediate transport across and/or penetrate and/or disrupt the blood-brain barrier. In preferred embodiments of the invention the above described approached to accessing the central nervous system are therefore disclaimed. In a preferred embodiments of the invention the medicament according to the invention do not comprise a component with a known ability to target or mediate targeting of cells within the central nervous system and/or a component with a known ability to act as vehicle for the passage over the blood-brain barrier.

Expressed in more exact terms said medicament or formulation does not comprise a component with a known ability to target the neuronal and/or glial cells of the brain.

Preferred embodiments thus pertain to the use of lysosomal alpha-mannosidase, wherein said medicament does not comprise lysosomal alpha-mannosidase in a form, which is capable of permeating the central nervous system/blood brain barrier. Accordingly, it is also preferred that the lysosomal alpha-mannosidase has not been modified in order to obtain carrier-mediated transport across the blood-brain barrier and that the lysosomal alpha-mannosidase has not been formulated so as to obtain disruption of the blood-brain barrier.

In equally preferred embodiments the medicament comprises a lysosomal alpha-mannosidase and does not comprise
a) a vehicle or vector, such as a peptide or polypeptide, for delivery of lysosomal alpha-mannosidase into the central nervous system, and
b) a component with a known ability to cause opening or disruption of the blood brain barrier, and
c) an intact cell

More specifically, it is an object of the present invention to provide the use of a lysosomal alpha-mannosidase for the preparation of a medicament, wherein said medicament does not comprise a vehicle selected from the group consisting of exogenous peptides and proteins, including the insulin fragment and peptides and proteins derived from virus or bacteria, polyamines, lipophilic moieties including phospholipids, antibodies or antibody fragments such as transferrin receptor antibodies and toxins.

Also, the invention pertains to the use of lysosomal alpha mannosidase wherein said preparation of a medicament does not comprise modification of said lysosomal alpha-mannosidase by attachment of polyamines.

The medicament according to the invention may comprise or consist of a lysosomal alpha-mannosidase, which is formulated in an isotonic solution. The term "isotonic solution" refers conventionally to a solution having the same osmotic pressure as blood. In the context of the present invention the solution may be but is not limited to a human isotonic solution. Presently preferred are formulation buffers comprising or consisting of 3.10-3.50 mM Na₂HPO₄, 0.4-0.6 mM NaH₂PO₄, 25-30 mM Glycine, 220-250 mM Mannitol, and water for injection. Equally preferred are formulation buffers comprising or consisting of 4.0-5.0 mM Sodium Citrate, 0.3-0.8 mM Citric Acid, 200-250 mM Mannitol, 3.0-5.0 mM Tween 80, and water for injection. Alternatively, the lysosomal alpha-mannosidase may be formulated in Phosphate buffered saline (0.01 M phosphate buffer + 0.145 M NaCl, pH=7.2).

As mentioned above, the use of intact cells may provide means for delivery of substances across the blood-brain barrier. The present invention, however relates to a medicament which does not comprise an intact cell.

Accordingly, a further preferred embodiment of the invention pertains to the use of lysosomal alpha-mannosidase, wherein said preparation of a medicament does not involve the use of a cell-mediated delivery system. In particular the present invention does not rely on the use of an intact cell selected from the group consisting of a transduced autologous cell, such as a transduced fibroblast or a peripheral blood lymphocyte and polymer encapsulated cell line capable of secreting alpha-mannosidase into the cerebrospinal fluid.

It is to be expected that the administration of lysosomal alpha-mannosidase will result in decreased accumulation of neutral oligosaccharides in a large variety of cells within the central nervous system. In preferred embodiments of the invention, however, said cells within one or more regions of the central nervous system comprise neuronal cells and/or glial cells. In addition, said cells may comprise extraneural cells present within the central nervous system.

The brain comprises four major parts, the cerebrum, the diecephalon, the brainstem and the cerebellum. It is to be understood that administration of alpha-mannosidase affects the storage of oligosaccharides in one or more of these parts. In further preferred embodiments of the invention said one or more regions the central nervous system comprise one or more regions within the cerebrum, diencephalon, brainstem and/or cerebellum.

It is likely that reduction in the accumulation of neutral mannose-rich oligosaccharides within certain specific regions of the four above-mentioned brain parts is of particular relevance with respect to reducing the clinical manifestations of alpha-mannosidosis. In more particular embodiments of the invention the accumulation of oligosaccharides is reduced within one or more regions within the cerebrum being selected from the group comprising one or more regions within the cerebral cortex, one or more regions within the subcortical nuclei, and one or more regions within the limbic system.

A number of sensory and/or motor functions have been ascribed to various specific regions within the cerebral cortex. Within the Frontal Lobe, which is generally motor in function, the Precentral Gyrus (Primary Motor Cortex) is responsible for control of fine movement, the Motor Association Cortex is responsible for Integration of information for complex movements, the Speech-Motor Cortex is involved in production of speech; (Broca's area), and the Pre-Frontal Cortex is involved in decision making; direction of attention.

Within the Parietal Lobe, which is generally sensory in function, the Postcentral Gyrus (Primary Somatosensory Cortex) is involved in perception of sensation from skin (Cutaneous) and muscle (proprioceptive), the Sensory Association Cortex is involved in integration of sensory signals; direction of sensory attention, the Left Association Cortex is involved in production of meaning to language; (Wernicke's area), and the Right Association Cortex is involved in production of meaning to spatial relationships

Within the Occipital Lobe, which is generally sensory in function, the Visual Cortex is involved in perception of visual sensation the Visual Association Cortex is involved in perception of visual content and meaning

Within the Temporal Lobe, which is generally sensory in function, the Primary Auditory Cortex is involved in perception of auditory sensation, the Auditory Association Cortex is involved in perception of auditory content and meaning; it extends into parietal cortex Gustatory and Olfactory Cortex which are involved in perception of gustatory and olfactory sensation, the Insular Cortex located at the deep extent of lateral sulcus is involved in providing knowledge about outcome of events.

Other regions include the Parahippocampal Gyrus, which overlies the hippocampus, the Hippocampus, which is involved in formation of long-term memory and the Amygdala, which is involved with feeling of emotion.

In even more particular embodiments of the invention, the levels of oligosaccharides are reduced in one or more regions within the cerebral cortex being selected from the group comprising the frontal lobe, the parietal Lobe, the temporal lobe and the occipital Lobe.

In other particular embodiments of the invention, the levels of oligosaccharides are reduced in one or more regions within the diencephalon being selected from the group comprising the thalamus and the hypothalamus.

The thalamus is a component of the diencephalon buried deep within the core of the brain. Richly and reciprocally connected with cortical structures, thalamic nuclei function both to relay and modulate the transmission of information throughout the brain.

The thalamus comprises the following main parts: The anterior nuclear group, the midline nuclear group, the medial dorsal nucleus, the intralaminar nuclear group, the lateral nuclear group, the ventral nuclear group, the geniculate bodies, the posterior nuclear complex, the thalamic reticular nucleus, the thalamic fiber tracts, and the stratum zonale of thalamus.

The limbic system is a group of brain structures that are involved in various emotions such as aggression, fear, pleasure and also in the formation of memory. The limbic system also affects the endocrine system and the autonomic nervous system. It consists of several structures located around the thalamus, just under the cerebrum: The hippocampus, which is involved in the formation of long-term memory, the amygdala, which is involved in aggression and fear, the cingulate gyrus, the fornicate gyrus, the archicortex and the hypothalamus, which controls the autonomic nervous system and regulates blood pressure, heart rate, hunger, thirst, and sexual arousal. Connected to the pituitary gland and thus regulates the endocrine system.

Neurological functions located in the brainstem include those necessary for survival (breathing, digestion, heart rate, blood pressure) and for arousal (being awake and alert). Most of the cranial nerves come from the brainstem. The brainstem is the pathway for all fiber tracts passing up and down from peripheral nerves and spinal cord to the highest parts of the brain. In additional particular embodiments of the invention the levels of oligosaccharides are reduced in one or more regions within the brainstem being selected from the group comprising the midbrain, the pons and the medulla oblongata.

The medulla oblongata functions primarily as a relay station for the crossing of motor tracts between the spinal cord and the brain. It also contains the respiratory, vasomotor and cardiac centers, as well as many mechanisms for controlling reflex activities such as coughing, gagging, swallowing and vomiting. The midbrain serves as the nerve pathway of the cerebral hemispheres and contains auditory and visual reflex centers. The pons is a bridge-like structure, which links different parts of the brain and serves as a relay station from the medulla to the higher cortical structures of the brain. It contains the respiratory center.

The function of the cerebellum, which includes the cerebellar cortex, is to facilitate the performance of movements by coordinating the action of the various participating muscle groups. In other particular embodiments of the invention, the levels of oligosaccharides are reduced in one or more regions within the cerebellum comprising the cerebellar cortex.

A primary advantage of the use of recombinant lysosomal alpha-mannosidase according to the present invention relates to fact that effects of the treatment seen in brain may combine with effects in cells that are not part of the central nervous system. Thereby, the administration of exogenous lysosomal alpha-mannosidase is likely to correct most if not all of the clinical manifestations of alpha-mannosidosis. In preferred embodiments, the invention thus pertains to the use of a medicament which further reduces the levels of neutral mannose-rich oligosaccharides in cells of neurological tissues, which are not within the central nervous system. In this embodiment of the invention, said medicament further comprises a component, which is capable of targeting cells of neurological tissues, which are not within the central nervous system. In particular the use of this medicament results in the correction of lysosomal storage in cells, which are not within the central nervous system, comprising the most prominent cells of the peripheral nervous system. These comprise neuronal cells and/or Schwann cells.

The clinical manifestations of alpha-mannosidosis may be partly alleviated by the isolated effects of replacing the deficient alpha-mannosidase in visceral tissues. However, a central part of the concept, which forms the basis of the present invention, relates to the fact that the beneficial effects of the therapy on lysosomal storage in the central nervous system is secondary to and/or dependent on a reduction in the lysosomal storage in tissues outside the central nervous system. Accordingly, it is an important characteristic of the invention that said medicament is also used to obtain a reduction in the levels of neutral mannose-rich oligosaccharides in cells within non-neurological tissues and that the medicament comprises a component capable of targeting cells within such tissues.

In this context, the effects of exogenous alpha-mannosidase in viscereal tissues are believed to be of primary importance. According to a preferred embodiment of the invention the medicament therefore comprises a component, which is capable of targeting neurological and/or non-neurological tissues cells outside the central nervous system. In an even more preferred embodiment said neurological and/or non-neurological tissues outside the central nervous system comprise visceral tissues selected from the group comprising liver, spleen, kidney and heart.

It is implicated that the exogenous alpha-mannosidase can be delivered to target cells in relevant tissues outside the central nervous system by use of for instance receptor mediated endocytosis. Preferably, the alpha-mannosidase is administered over cellular membranes of such target cells taking advantage of a mannose- or mannose-P-6-receptor-mediated uptake. Accordingly, the above mentioned factor possessing targeting abilities is preferably mannose-6-phosphate.

It is contemplated that any mammalian alpha-mannosidase may be used for replacing the deficient enzyme in a subject. However, when the subject is a human the use of a human lysosomal alpha-mannosidase will be preferred in order to, for instance, minimise the occurrence of antigenic reactions. Accordingly, preferred embodiments of the invention pertains to the use of a lysosomal alpha mannosidase, which is a human lysosomal alpha mannosidase.

In a most preferred embodiment, the lysosomal alpha mannosidase comprises the amino acid sequence of SEQ ID NO.: 1.

For practical and economical reasons it is preferred that the alpha-mannosidase is produced recombinantly. Furthermore, it is to be expected that for a lysosomal hydrolase purified from tissue that the enzyme has a low content of mannose-6-phosphate. Thus, by recombinant production it will also be possible to obtain a preparation of the enzyme wherein a large fraction contains mannose-6-phosphate. Recombinant production may be achieved after transfection of a cell using a nucleic acid sequence comprising the sequence of SEQ ID NO: 2.

The alpha-mannosidase is preferably made in a mammalian cell system in order to provide a glycosylation profile, which ensures efficient receptor mediated uptake in cells of for instance visceral organs of the body. In particular, it has been found that production of the enzyme in CHO, COS or BHK cells ensures adequate post-translational modification of the enzyme by addition of mannose-6-phosphate residues. In addition a correct sialylation profile is obtained. Correct sialylation is known to be important in order to prevent uptake by the liver, because of exposed galactose residues.

In even more preferred embodiments the mammalian cell system is therefore selected from the group comprising CHO, COS cells or BHK cells (Stein et al. J Biol Chem.1989, 264, 1252-1259). It may further be preferred that the mammalian cell system is a human fibroblast cell line.

In a most preferred embodiment, the mammalian cell system is a CHO cell line.

It follows that equally preferred embodiments of the invention pertains to the use of a preparation of lysosomal alpha-mannosidase wherein a fraction of said preparation consists of lysosomal alpha mannosidase having one or more N-linked oligosaccharides carrying mannose 6-phosphate groups.

It is further preferred that a fraction of a preparation of said lysosomal alpha-mannosidase is capable of binding to mannose 6-phosphate receptors.

The ability of the enzyme to bind to mannose-6-phosphate receptors may be determined in an *in vitro* assay as described in example 1 of the present application. Here, binding of the enzyme to a MPR affinity 300 Matrix provides a measure of its ability to bind to mannose-6-phosphate receptors. In a preferred embodiment of the invention binding of the enzyme to mannose-6-phosphate receptors occurs *in vitro.*

In more preferred embodiments of the invention this fraction corresponds to from 1 to 75% of the activity of a preparation of lysosomal alpha-mannosidase, such as from 2 to 70%, such as from 5 to 60%, such as from 10 to 50% such as from 15 to 45%, such as from 20 to 40%, such as from 30 to 35%.

Accordingly, it is preferred that the lysosomal alpha-mannosidase has a content of mannose 6-phosphate residues allowing mannose 6-phosphate dependent binding of from 2 to 100%, 5 to 95%, 10 to 90%, 20 to 80%, 30 to 70% or 40 to 60% of the amount of enzyme to a Man-6-P-receptor matrix. At present, the degree of phosphorylation has been analysed in several batches of enzyme and, typically, from 30 to 45% of the enzyme is phosphorylated and binds the affinity matrix.

It is further preferred that a fraction constituting from 2 - 100%, 5 - 90%, 10 - 80%, 20 - 75%, 30 - 70%, 35 - 65% or 40 - 60% of the amount of said lysosomal alpha-mannosidase binds to the Man-6-P-receptor with high affinity. Theoretically, two mannose 6-phosphate groups must be positioned close to each other in order for the enzyme to bind a Man-6-P-receptor with high affinity. Recent observations suggest that the distance between the phosphorylated mannose residues must be 40 Å or less in order to obtain high affinity binding. In the human lysosomal alpha-mannosidase according to SEQ ID NO: 1 the two mannose 6-phosphate residues may be situated at the asparagines residues in positions 367 and 766. Accordingly, it is preferred that the medicament according to the present invention comprises lysosomal alpha-mannosidase, a fraction of which carries mannose 6-phosphate groups at both of these asparagine residues.

It is to be understood that various different means of administration can be employed for delivering the enzyme. These include parenteral including intravenous, intra-arterial, subcutaneous, intraperitoneal or intramuscular injection or infusion. Since administration is likely to be repeated on a regular basis the use of simple techniques for the administration is preferred. In a currently most preferred embodiment of the invention, alpha mannosidase is used for the preparation of a medicament, which is for intravenous administration. In particular, administration by injection directly into the central nervous system is considered not to be suitable since administration via this route cannot be performed by a lay-person.

It will of course be possible to co-administer the medicament, which is prepared by the use of the alpha-mannosidase according to the invention with a composition comprising a component with a known ability to target or mediate targeting of the central nervous system. In a much-preferred embodiment of the present invention such co-administration is neither necessary nor desired and is not to be included in the treatment scheme. This embodiment pertains to the use according to the invention, wherein said medicament is for administration without supplementary administration of a composition comprising a component with a known ability to target the central nervous system.

It appears that the medicament is to be administered in doses, wherein the amount of lysosomal alpha-mannosidase is sufficient to correct the defects in lysosomal storage. It appears from previous studies on related lysosomal disorders, that a complete substitution of the deficient enzyme, so that levels corresponding to those seen in healthy individuals are reached, may not be necessary. In order to minimise the cost of the therapy and possibly also reduce putative adverse effects the aim should be to use as small amounts of the enzyme as possible. In addition, it appears possible to "tailor" the treatment including the amounts administered and the frequency of administration according to the profile of the individual subject. Therefore, in preferred embodiments of the present invention, the medicament is administered in an amount, which is within the range of 1 to 400 mU lysosomal alpha-mannosidase per gram body weight of the subject to be treated, such as within the range of 2 to 350, 5 to 300, 7,5 to 200, 10 to 200, 15 to 150, 20 to 100, 25 to 75, 30 to 60, 35 to 50 or 40 to 45 mU lysosomal alpha-mannosidase per gram body weight,

In a currently more preferred embodiment of the invention the medicament is administered in an amount of 250 mU lysosomal alpha-mannosidase per gram body weight. In an equally preferred embodiment of the invention, the medicament is administered in an amount of 50 mU lysosomal alpha-mannosidase per gram body weight.

The specific activity of the lysosomal alpha-mannosidase may be a crucial factor influencing the ability of the wherein said lysosomal alpha-mannosidase has a specific activity of 5 - 100 U/mg, such as from 10 - 90 U/mg, 15 - 80 U/mg, 20 - 70 U/mg, 25 - 65 U/mg, 30 - 60 U/mg or 35 - 55 U/mg.

In the enzyme preparation used in the present invention, a fraction of the amount of lysosomal alpha-mannosidase is present in the form of a 260 kDa dimer composed of 130 kDa monomers of an amino acid sequence having at least 75%, 80%, 85%, 90%, 95%, 97%, 98% or 99% sequence identity to SEQ ID NO: 1. When analysed under reducing conditions, however, a variable fraction of the amount of enzyme appears to have been processed into the 70 and 55 kDa forms. This indicates that a fraction of the amount of the lysosomal alpha-mannosidase has been partly cleaved but is still held together in the 260 kDa form by disulfide bridges. Both the 130 kDa form and the processed forms are active, but it is preferred that a large fraction of the amount of enzyme is present as dimers of the 130 kDa precursor form when administered to the subject. Preferably, at least 30%, 35%, 40%, 45%, 50%, 55%, 60%, 65%, 70%, 75%, 80%, 85%, 90%, 95% or 100% is present as a dimer of the 130 kDa form. When the enzyme is subsequently taken up into the lysosomes it is processed into the 15, 42 and 70 kDa peptides. The 70 kDa peptide is further processed to three smaller peptides that are still kept together by disulfide bridges.

As enzyme replacement therapy is only expected to generate transient effects, it further appears that the medicament may be administered in a frequency ranging from several daily injections to injections once or twice every week or month. In preferred embodiments the medicament may be administered in a frequency corresponding to 1 to 5 daily injections, 1 to 5 weekly injections, 1 to 5 injections every 2 weeks or 1 to 5 injections every month. Alternatively, the frequency may correspond to one injection every 1, 2, 3, 4, 5, 6, 7, 8, 9, 10, 11, 12, 13, or 14 days.

In equally preferred embodiments the medicament is administered by repeated intravenous injection on a daily basis, by repeated intravenous injection every 2, 3, 4, 5, or 6 days, or by repeated intravenous injection on a weekly, bi-weekly or monthly basis.

In a currently preferred embodiment of the invention the amount of lysosomal alpha-mannosidase is administered once every 3 to 4 days.

Administration of the medicament according to the present invention by intravenous injection leads to only insignificant increases in the amount of lysosomal alpha-mannosidase present within the central nervous system. While not being bound by this theory it appears that the reduced levels of neutral oligosaccharides may be secondary to and a direct or indirect result of the increased levels of enzyme in the peripheral tissue and visceral organs. Since the lysosomal alpha-mannosidase appears to be rapidly cleared from the circulation, it appears critical that the amounts of enzyme administered are such that the levels of circulating enzyme remain adequately increased for an amount of time allowing for cellular uptake of appropriate amounts of enzyme via the Man-6-P receptor or, possibly, also via other presently unknown transport mechanisms. In addition, levels of enzyme within the visceral organs and the peripheral tissue must remain adequately increased for an amount of time sufficient to cause a reduction in the neutral sulfatides not only within the organs or tissue but also within the central nervous system. Accordingly it is preferred that the amount of lysosomal alpha-mannosidase administered is such that:
a) effective levels of the enzyme are sustained in circulation for not less than 5 - 30 minutes such as not less than 5, 10, 15, 20, 25, or 30 minutes, and/or
b) effective levels of the enzyme are sustained in visceral organs, including the kidney, spleen and heart, for not less than 1, 2, 3, 4, 5, 6, 7, 8, 9, 10, 12, or 24 hours, and/or
c) effective levels of the enzyme are sustained in the liver for not less than 6, 12 or 24 hours or 1, 2, 3, 4, 5, 6, 7, 8, 9, or 10 days,
subsequent to injection, such as intravenous injection, of said medicament.

In the present context the term "effective levels" refer to levels of lysosomal alpha-mannosidase that are increased by at least two fold, at least 5 fold, at least 10 fold, at least 20 fold or at least 50 fold compared to the enzyme levels seen in serum or organ extracts from untreated subjects when analysed in an enzyme assay as described in example 2 of the present application.

Preferably, the enzyme once administered to said subject will lead to a reduction in the levels of intralysosomal oligosaccharides preferably corresponding to 20%, more preferably 30%, even more preferably 40%, still more preferably 50%, yet more preferably 60%, most preferably more than 70%.

In particular, the amount of lysosomal alpha-mannosidase administered is preferably such that:
a) the amount of neutral oligosaccharides in visceral organs including the liver, spleen, kidney and/or heart is reduced by 10 - 80%, 15 - 85%, 20 - 90%, 25 - 95% or 30 - 100%, such as at least 10, 15, 20, 25, 30, 35, 40, 45, 50, 55, 60, 65, 70, 75, 80, 85, 90 or 95% over 2, 4, 6, 8, 10, 12 18 or 24 hours, as compared to the levels before treatment or the levels seen in untreated subjects, and/or
b) b) the amount of neutral oligosaccharides in visceral organs including the liver, spleen kidney and/or heart is reduced by 40 - 80%, 45 - 85%, 50 - 90%, 55 - 95% or 60 - 100%, such as at least by 40, 45, 50, 55, 60, 65, 70, 75, 80, 85, 90 or 95% over a period of 1 - 4, 2 - 6 or 4 - 8 days , and/or
c) c) the storage of neutral oligosaccharides in the liver is reduced over a period of 1 to 12, 2 - 10 or 4 - 8 days as determined by a reduced presence of storage vacuoles within the sinus endothelial cells, Kupffer cells and hepatocytes
subsequent to injection, such as intravenous injection, of said medicament.

Central to the present invention is the observation that a reduction in the levels of neutral oligosaccharides in the central nervous system is seen only after repeated administration of lysosomal alpha-mannosidase. Therefore, it may be preferred that the amount of lysosomal alpha-mannosidase administered is such that:
a) effective levels of the enzyme are sustained in circulation, and/or
b) effective levels of the enzyme are sustained in visceral organs, including the kidney, spleen and heart, and/or
c) effective levels of the enzyme are sustained in the liver,
by repeated injection, such as intravenous injection, of the medicament on a daily basis, by repeated injection, such as intravenous injection, every 2, 3, 4, 5, or 6 days, or by repeated injection, such as intravenous injection, on a weekly, bi-weekly or monthly basis.

It may be equally preferred that the amount of lysosomal alpha-mannosidase administered is such that
a) the storage of neutral oligosaccharides in kidney and heart is fully corrected or fully corrected at least over a period of 1, 2, 3, 4, 5, 6, 7, 8, 9, 10, 11, 12, 13 or 14 days, and/or
b) the amount of neutral oligosaccharides in the spleen is reduced by at least 10, 20, 30, 40, 50, 60, 70, 80, 90 or 100% or so reduced at least over a period of 1, 2, 3, 4, 5, 6, 7, 8, 9, 10, 11, 12, 13 or 14 days, and/or
c) the storage of neutral oligosaccharides in the liver and the kidney is reduced or reduced over a period of 1, 2, 3, 4, 5, 6, 7, 8, 9, 10, 11, 12, 13 or 14 days as determined by a reduced presence of storage vacuoles within the sinus endothelial cells, Kupffer cells and hepatocytes of the liver and in the tubular epithelia of the kidney
by repeated injection, such as intravenous injection, of the medicament on a daily basis, by repeated injection, such as intravenous injection, every 2, 3, 4, 5, or 6 days, or by repeated injection, such as intravenous injection, on a weekly, bi-weekly or monthly basis.

The reduction of stored oligosaccharides in the brain may result, at least in part, from the clearance of storage material from brain endothelia and from the meninges, which may improve the microcirculation and the flow of the cerebrospinal fluid. A much preferred embodiment of the present invention thus pertains to the use of lysosomal alpha-mannosidase for the preparation of a medicament for reducing the intracellular levels of neutral mannose-rich oligosaccharides in cells within one or more regions of the brain endothelia and/or the meninges.

It is to be understood that the medicament may have the characteristics describe above. Thus, in a preferred embodiment of this aspect of the invention, said medicament does not comprise a component with a known ability to target or mediate targeting of the central nervous system and/or a component with a known ability to act as vehicle for the passage over the blood-brain barrier.

Again it is preferred that the method comprises administering said medicament of alpha-mannosidase by a route other than intracerebroventricular, spinal, intrathecal or intracranial administration.

Particularly preferred is administration of the medicament by intravenous or subcutaneous injection.

Co-administration of the medicament according to the present invention with a component with a known ability to target or mediate targeting of the central nervous system and/or a component with a known ability to act as vehicle for the passage over the blood-brain barrier is neither necessary nor desired and is not to be included in the treatment scheme. It is thus preferred that the administration of said medicament is not supplemented by the administration of a composition comprising a component with a known ability to target the central nervous system.

According to a recent publication, intrathecal injection, that is injection directly into the cerebrospinal fluid) of recombinant human alpha-L-iduronidase (rhIDU) can reduce corbohydrate storage in brain tissue in a canine model of mucopolysaccharidosis (MPL) (Kakkis, 2003). While intravenous administration of the medicament according to the present invention is by far preferred, the observations of Kakkis suggest that such administration may at least in some cases be combined with other treatments, including other routes of administration.

The invention may thus provide the use of a lysosomal alpha-mannosidase for the preparation of a medicament for intravenous administration as well as for administration by intrathecal or spinal injection, wherein the combined intravenous and intrathecal administration results in a reduction in the galactosyl sulphatide levels in target cells within the peripheral nervous system and in target cells within the central nervous system in a subject.

Further, the invention may provide the use of a lysosomal alpha-mannosidase for the preparation of a medicament for intravenous administration, where intravenous administration of the medicament results in a reduction in the galactosyl sulphatide levels in target cells within the peripheral nervous system and in target cells within the central nervous system.

In yet another aspect, a method for the preparation of recombinant human alphamannosidase is provided, the method comprising a) introducing, into a suitable vector, a nucleic acid fragment comprising a DNA fragment which codes for the amino acid sequence shown SEQ ID NO.:1; b) transforming a cell with the vector obtained in step a);

c) culturing the transformed host cell under conditions facilitating expression of the nucleic acid sequence; d) recovering the expression product from the culture. The method may further comprise a series of one or more purification steps.

In particular, a method is provided, wherein the nucleic acid fragment comprises the 3066 basepair EcoRI - XbaI fragment of the DNA fragment shown in SEQ ID NO 2.

The production of recombinant alpha-mannosidase, which is mannose-6-P comprises one or several or all of the following general steps (an outline):

### A. Synthesis of rhLAMAN

### Cloning of specific rhLAMAN

### B. Transfection

2-10 µg rhLAMAN hybrid vector DNA is used for transfection by phosphate precipitate/ glycerol shock methodology, into mammalian cells (e.g. CHO, COS cells or BHK cells). Transfection might also be done with an electric shock methodology.

### C. Expression of rhLAMAN

Synthesis of the active protein and the mannose-6-P modification of said protein is done during the expression in the mammalian cell system.

### D. Purification of rhLAMAN

recombinant human alpha-mannosidase is purified using a 6 step procedure - see example 1.

### E. Test system for mannose-6-P receptor mediated uptake

The ability of produced recombinant alpha-mannosidase to be active in a mannose-6-P receptor mediated uptake is tested by incubating alpha-mannosidase with normal fibroblasts or fibroblasts from alpha-mannosidosis patients. Uptake into cells is assayed by increased alpha-mannosidase activity.

In a currently preferred embodiment the alpha-mannosidase is produced in a mammalian cell system selected from the group comprising BHK, COS and CHO cells. One of the critical parameters of the method is the glycosylation profile of the alpha-mannosidase in the final preparation. The glycosylation profile is a major determinant of the corrective activity of the enzyme in vivo via its effects on receptor mediated endocytosis and clearance of the enzyme form the blood stream. When using the above mentioned preferred mammalian cell systems it has been found that alpha-mannosidosis with an appropriate glycosylation profile is produced; in particular use of CHO cells leads to the production of alpha-mannosidase with an adequate content of mannose-6-phosphate. In the examples of the present application the ability of the alpha-mannosidase to bind to an MPR 300 column provides a measure of its content of mannose-6-phosphate. The mannose-6-phosphate containing alphamannosidase is secreted into the medium and purification of the enzyme may be facilitated by the use of ammonium salts (NH₄Cl) in the fermentation step.

Currently, a factor, which must be taken into consideration when isolating and purifying the enzyme, is the occurrence of dephosphorylation during isolation. While preparation of human alpha-mannosidase have been obtained in which a fraction corresponding to 40% or more of the enzyme activity was able to bind to the MRP 300 column this fraction may be as low as a few per cent. In order to reduce dephosphorylation during the processing steps the presence of one or more phosphatase inhibitors appears crucial. Such phosphatase inhibitors are available from commercial sources.

An important aspect of the invention relates to the use of a recombinant alpha-mannosidase to for the preparation of a medicament for the treatment of alpha-mannosidosis. In particular, it is preferred that the alpha-mannosidase is a recombinant human alpha-mannosidase.

The disease, which is the target for the inventive method is alpha-mannosidosis, and therefore the catalyst is alpha-mannosidase or an enzymatically equivalent part or analogue thereof. It is most preferred that the catalyst is a recombinant form of the human alpha-mannosidase enzyme or of the enzymatically equivalent part or analogue thereof, since recombinant production will allow large-scale production which, with the present means available, does not seem feasible if the enzyme would have to be purified from a native source.

Preferably, the alpha-mannosidosis is made by recombinant techniques. In a further embodiment, the alpha-mannosidosis is human (hLAMAN) and still more preferred mature human alpha-mannosidase (mhLAMAN) or a fragment thereof. The fragment may be modified, however the active sites of the enzyme should be preserved.

It is to be expected that, in preparations of alpha-mannosidosis according to the present invention, one fraction of the enzyme is represented by its precursor form, while other fractions represent the proteolytically processed forms of approximately 55 and 70 kDa. In general, the target cell is a cell wherein the enzymatic activity, such as the alpha-mannosidase activity is insufficient for the optimal function of the cell. Insufficient activity of alpha-mannosidase may be measured by one or more of the parameters selected from monitoring incresed levels of mannose-rich oligosaccharides in urine, analysis of alpha-mannosidase activity in material from a subject such as in leukocytes and/or in skin fibroblasts, presence of clinical symptoms or increase in rate of development of clinical symptoms of alpha-mannosidosis.

A significant feature of insufficient alpha-mannosidase activity is a cell wherein a massive intracellular accumulation of mannose-rich oligosaccharides is present. Naturally, such cell is a target cell according to the present invention. The target cell may also be a cell of the nervous system. In addition, target cells for delivering the enzyme also includes one or more cell types selected from human monocytes, human fibroblasts, human lymphocytes, human macrophages.

An increased activity of the alpha-mannosidase may be used as a parameter for evaluating the success of a treatment schedule. The activity be measured by one or more of the parameters selected from monitoring incresed levels of mannose-rich oligosaccharides in urine, analysis of alpha-mannosidase activity in material from the patient such as in leukocytes and/or in skin fibroblasts, presence of clinical symptoms or increase in rate of development of clinical symptoms of alpha-mannosidosis.

It is a very important aspect of the invention to perform the treatment of a possible enzyme defect prenatally. In a further aspect of the invention, the cellular membrane is the fetal-maternal barrier (placenta). As is the case for the blood brain barrier it may be an important characteristic of the present invention that actual delivery of the enzyme over the fetal-maternal barrier or disruption of the fetal-maternal barrier is unnecessary and undesired.

With respect to the above description of the various aspects of the present invention and of the specific embodiments of these aspects it should be understood that any feature and characteristic described or mentioned above in connection with one aspect and/or one embodiment of an aspect of the invention also apply by analogy to any or all other aspects and/or embodiments of the invention described.

When, in the present application, an object according to the present invention or one of its features or characteristics is referred to in singular this also refers to the object or its features or characteristics in plural. As an example, when referring to "a cell" it is to be understood as referring to one or more cells.

Throughout the present specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

### LEGENDS TO FIGURES

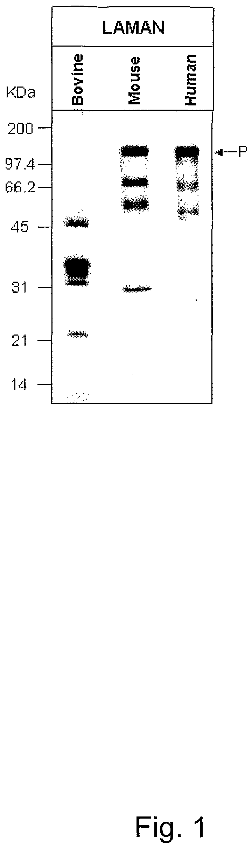

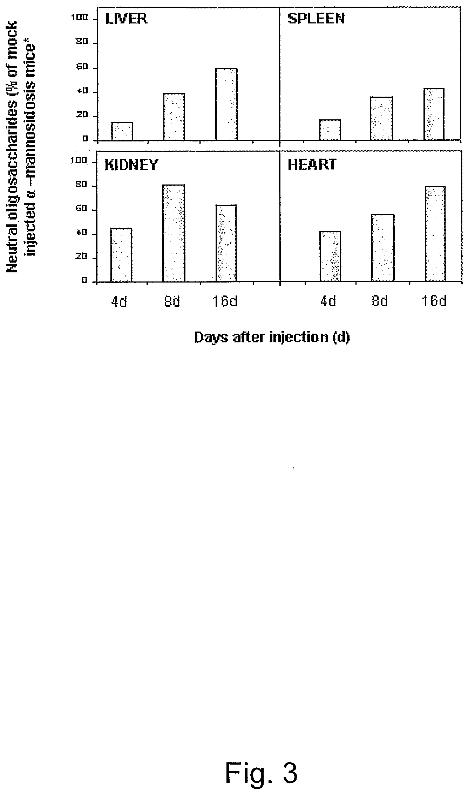

Fig. 1: Polypeptide pattern of bovine, mouse and human LAMAN
   10 µg of LAMAN purified from bovine kidney or the secretions of cell overexpressing mouse or human LAMAN were separated by SDS-PAGE and stained with Coomassie Blue. The 130 kDa polypeptides seen in mouse and human LAMAN correspond to the precursors and the polypeptides ranging from 11 to 70 kDa to proteolytically processed forms of LAMAN. The 46-48 kDA polypeptide in bovine LAMAN represents partially processed intermediates.
Fig. 2: Thin-layer chromatography of neutral oligosaccharides in liver, spleen, kidney and heart.
   The fraction of neutral oligosaccharides from equal amounts of tissues was separated by thin-layer chromatography. Lane 1 contains the sample from control mice, lane 2 from mock-injected LAMAN mice, lane 3, 4 and 5 the samples from α-mannosidosis mice injected with 100 mU mouse LAMAN per g body weight and killed after 2, 4 and 10 h, respectively (see also Table 1). Oligosaccharides were detected with orcinol/ sulphuric acid. Mass spectrometry and sensitivity to jack bean α-mannosidase demonstrated that the oligosaccharides M2 to M9 contain 2 to 9 mannose residues and a single N-acetylglucosamine residue at the reducing terminus. The oligosaccharides were quantified by densitometry. For calculation of the storage the values for neutral oligosaccharides in α-mannosidosis mice were corrected for the neutral oligosaccharides in control mice and expressed as percentage of that in mock-injected α-mannosidosis mice. The storage in LAMAN injected α-mannosidosis mice was expressed as percentage of that in mock-injected α-mannosidosis mice. Mass spectrometry and sensitivity to jack bean α-mannosidase showed that the oligosaccharides M2 to M9 contain 2 to 9mannose residues and a single Nacetylglucosamine residue at the reducing terminus.
Fig. 3: Neutral oligosaccharides in tissue extracts of α-mannosidosis mice injected with 100 mU mouse LAMAN per g body weight.
   The mice were killed 4, 8 and 16 days after injection and the neutral oligosaccharides in extracts of liver, spleen, kidney and heart were quantified by densitometry after separation by thin layer chromatography (see legend of Fig. 2).
Fig. 4: Clearance of LAMAN from serum
   LAMAN of bovine (□), mouse (■) or human (○) origin, 50 mU/g body weight, was injected into the tail vein of 8 weeks old α-mannosidosis mice. Blood was collected from the retroorbital plexus 5 to 65 min after injection. Each value represents the mean of two animals. The range is indicated by bars where it exceeds the size of the symbols.

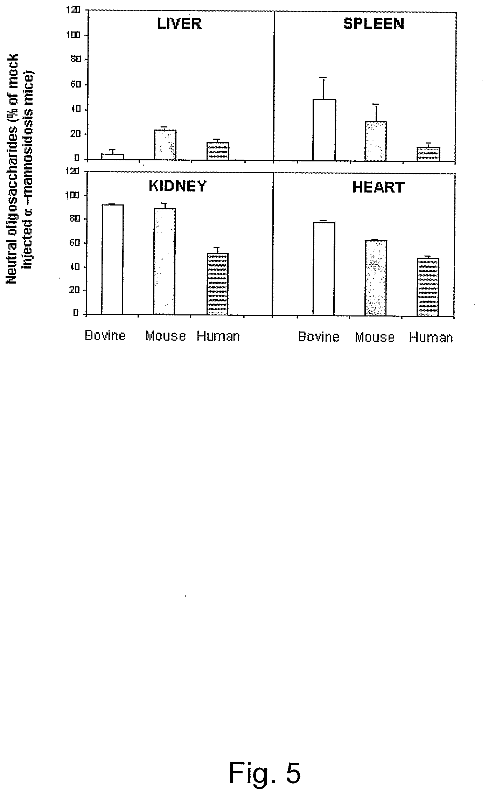

Fig. 5: Corrective effect of bovine, mouse and human LAMAN on the storage of neutral oligosaccharides
   Mice received 50 mU LAMAN per g body weight (see Fig. 3) 2 days prior tissue analysis. Neutral oligosaccharides were quantified as in Figs. 2 and 3. The bars indicate the range observed in two independent mice.

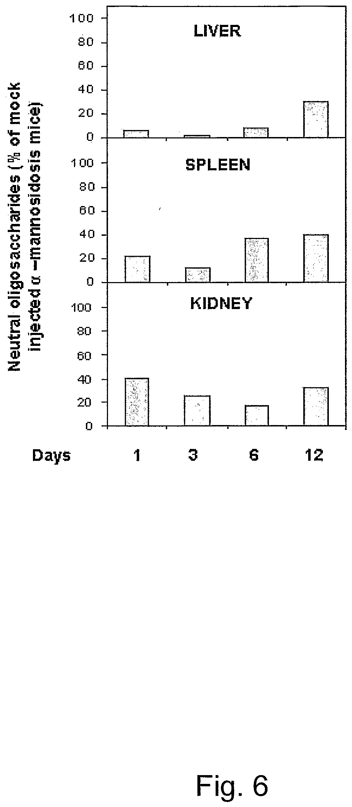

Fig. 6: Neutral oligosaccharides in tissue extracts of α-mannosidosis mice after injection of a single dose of 250 mU of human α-mannosidase per g body weight
   The mice were killed 1, 3, 6 and 12 days after injection. The neutral oligosaccharides in the tissue extracts of liver, spleen, kidney and heart were quantified by thin-layer chromatography and densitometry as in Figs. 1 and 2.

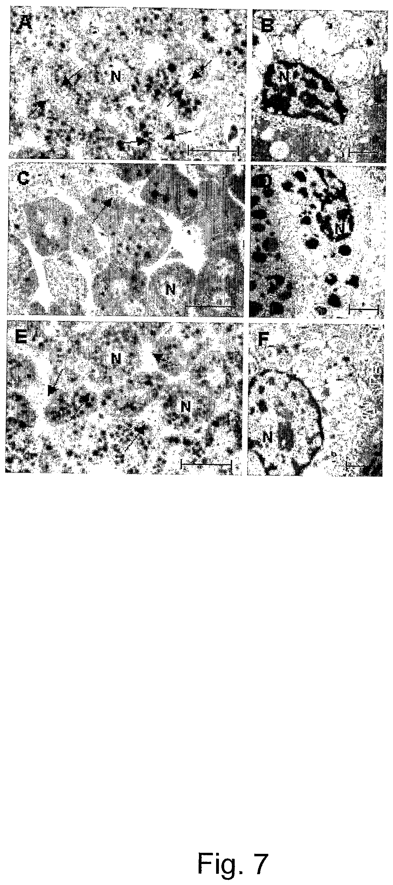

Fig. 7: Disappearance and reappearance of storage vacuoles in liver
   A, C, E, light microscopy (semithin sections); B, D, F, ultrastructure of Kupffer cells; A, B, mock-injected α-mannosidosis mouse (age 14 weeks). Hepatocytes show clear vacuoles along the bile canaliculi (A, arrows). The heavily vacuolated sinus wall cells are not clearly discerned a the light microscopic level (A) because of the extremely narrow cytoplasm bridges between the vacuoles as seen in (B). C, D, α-mannosidosis mouse, 1 day after injection of 250 mU human LAMAN per g body weight. Vacuoles have disappeared from hepatocytes (C). Very few vacuolated sinus wall cells are seen (C, arrow). The vacuoles in Kupffer cells are smaller than in the mock-treated animal and can contain an electron-dense matrix (D). E, F, α-mannosidosis mouse, 12 days after injection of 250 mU human LAMAN per g body weight. Vacuoles have reappeared in sinus wall cells (E, arrows) and less so in hepatocytes. In Kupffer cells (F) the vacuoles qualitatively resemble those in mock-injected mice. The dense inclusions in the hepatocytes shown in A, C, and E represent lipid droplets, which are equally encountered in the corresponding wild type mice (not shown). Bars represent 20 µm and 2 µm, respectively.

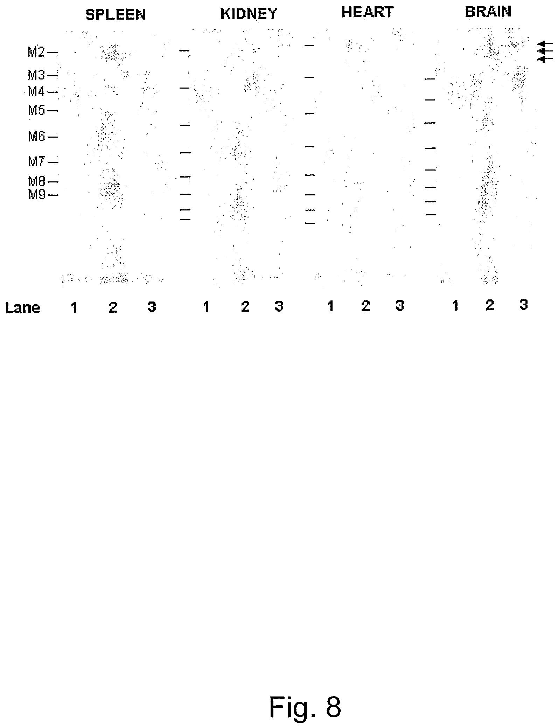

Fig. 8: Neutral oligosaccharides in tissue extracts of α-mannosidosis mice twice injected with 250 mU human LAMAN per g body weight
   Control (lane 1) and α-mannosidosis mice (lane 2 and 3) were injected with PBS (lane 1 and 2) or 250 mU human LAMAN per g body weight (lane 3) 7 and 3.5 days prior analysis. Neutral oligosaccharides were prepared and quantified as in Fig. 1 and 2. The migration of oligosaccharides composed of 2 to 9 mannose residues and a single N-acetylglucosamine residue (M2 to M9) is indicated. Arrows mark orcinol-positive material insensitive to LAMAN. The results in a duplicate set of mice (not shown) varied by less than 15%.

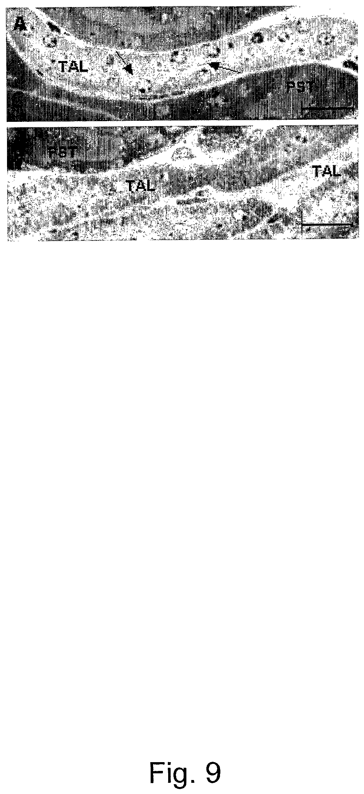

Fig. 9: Light microscopy of kidney in α-mannosidosis mice
   α-Mannosidosis mice were injected with PBS (A) or 250 mU human LAMAN per g body weight (B) 7 and 3.5 days prior killing. The outer stripe of the outer medulla is shown. Numerous clear vacuoles are seen in the thick ascending limb (TAL) of Henle's loop of the mock-injected mouse (A, arrows) in contrast to the LAMAN-treated mouse (B). The proximal straight tubules (PST) are free of pathological vacuoles in either animal. Bars represent 20 µm.

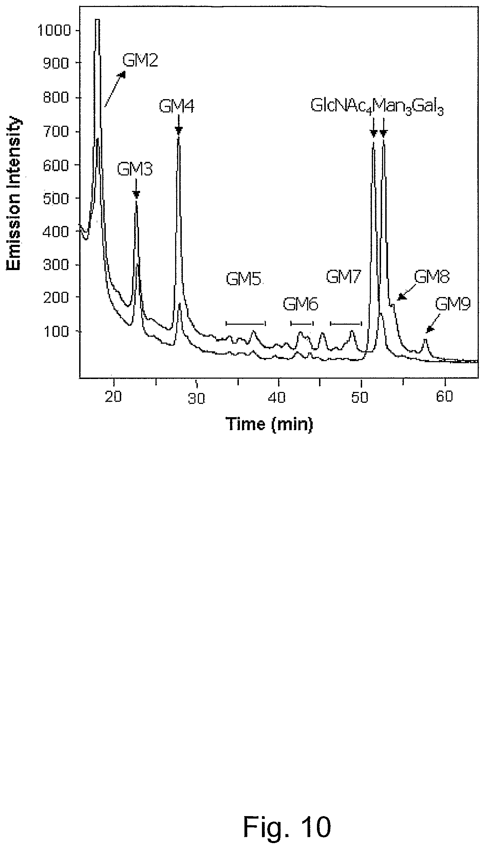

Fig. 10: HPLC-separation of 2-anthranilamide derivatized neutral oligosaccharides from brain
   Neutral oligosaccharides from brain of α-mannosidosis mice injected with PBS (upper scan) or 250 mU human LAMAN per g body weight (lower scan) 7 and 3.5 days prior killing were mixed with 220 pmol GlcNAc₄Man₃Gal₃ as an internal standard, derivatized with 2-anthranilamide and separated by HPLC. The position of the internal standard and of oligosaccharides composed of 2 to 9 mannose residues and a single N-acetylglucosamine residue (M2 to M9) are indicated.

### EXAMPLES

### Example 1: Production and characterisation of LAMAN

### Experimental procedures

### Expression and purification of recombinant human LAMAN in CHO cells

Human LAMAN cDNA isolated from HepG2 cDNA library and subcloned into an expression vector carrying a dihydrofolate reductase gene and the LAMAN cDNA under the control of the human CMV-promotor was expressed in Chinese Hamster Ovary (CHO) cells deficient in dihydrofolate reductase. The CHO cells were cultered in a two-compartment CELLine flask (Integra Biosciences Inc.) in serum free ExCell 302 medium (JRH Biosciences) supplemented with 20 nM methotrexate at 37°C in a humidified atmosphere containing 5% CO₂. The medium was diafiltrated using a Pellicon Biomax polysulphone filter with a 100 kDa cut off against 4 volumes 0.02 M Tris-HCl, pH 7.6. Ion-exchange chromatography was performed on DEAE-Sepharose FF (Amersham Pharmacia Biotech AB) using a NaCl gradient in 0.02 M Tris-HCl, pH 7.6. Active fractions were concentrated using an Amicon Centricon Plus-80 centrifugation filter with a 30 kDa cut off and subjected to gel filtration in a HiPrep 26/60 Sephacryl High Resolution column (Amersham Pharmacia Biotech AB) in 0.02 M Tris-HCl, pH 7.6, containing 0.15 M NaCl. After concentration the final preparation had a specific activity of 9-15 U/mg.

### Expression and purification of recombinant mouse LAMAN

The cDNA encoding the mouse LAMAN (16) was subcloned in the expression vector pMPSVEH (17). A polyhistidine tail (6 residues) had been added to the C-terminus part of the enzyme. Mouse embryonic fibroblasts deficient in the small and the large mannose 6-phosphate receptors (mpr-/- MEF) (18) were transfected and stably expressing clones were selected with 50 µl/ml hygromycin. For production of recombinant mouse LAMAN the cells were cultured in medium supplemented with 10% FCS in a humidified atmosphere containing 5% CO₂. The secreted recombinant mouse LAMAN was purified from conditioned medium using a three step procedure. In the first step the medium was dialyzed against 20 mM sodium phosphate buffer pH 7,8 containing 500 mM sodium chloride and then loaded onto a Probond column (Invitrogen). The retained enzyme was eluted with a gradient 0 to 0.35 M of imidazole (total volume 80 ml) in 20 mM sodium phosphate buffer pH 6.0 containing 500 mM sodium chloride. The LAMAN containing fractions were dialyzed against 10 mM sodium phosphate, pH 6.0 and loaded onto a DEAE-cellulose. The enzyme was eluted in a 0 to 0.25 M sodium chloride gradient (total volume 80 ml) in 10 mM sodium phosphate buffer. Finally mouse LAMAN was adsorbed to ConA-Sepharose (loading buffer 20mM Tris-HCl, pH 7.4, containing 1 mM MgCl₂, 1mM MnCl₂, 1 mM CaCl₂ and 0.5 M NaCl), and eluted with α-mannopyranoside (0.0-1.0 M) in the same buffer. The final preparation had a specific activity of 17-25 U/mg.

### Purification of bovine LAMAN

Bovine LAMAN was purified from kidney as described (19). The final preparation had a specific activity of 10 U/mg.

### Purification of human LAMAN

LAMAN was expressed in Chinese Hamster Ovary (CHO-) cells that was cultured in a two compartment CelLine flask, at 37°C in a humidified atmosphere with 5 % CO₂. The enzyme, obtained from the CHO-cells, was purified according to a purification procedure consisting of 4 steps:

### Diafiltration

The diafiltration was based on the TFF model, using a Pellicon Biomax polysulphone filter with a 100 kDa-cut off. The sample was diafiltrated against approximately 3 × sample volume with 0.02 M Tris-HCl, pH 7.6.

### Ion exchange chromatography

Ion exchange chromatography was performed using DEAE Sepharose flow filtration. Buffer was 0.02 M Tris-HCl pH 7.6. The gel was packed in a XK 16/20 column from Amersham Pharmacia Biotech AB. The eluation was performed with a gradient of NaCl.

### Concentration of sample

Prior to Gel filtration, the sample was concentrated to a protein concentration of ∼6 mg/mL and a final volume of 10 mL. This was carried out using a Amicon Centricon Plus-80 centrifugation filter with a 30 kDa cut off.

### Gel filtration

Gel filtration was carried out in a HiPrep 26/60 Sephacryl High Resolution column from Amersham Pharmacia Biotech AB. The sample was eluated with 0.02 M Tris-HCl supplied with 0.15 M NaCl, pH 7.6.

Example of a LAMAN purification is summarized in the table below. Normally Yield is in the range of 30 - 50 % and specific activity 9 - 15 U/mg.

### Summary of the LAMAN purification

| Purification step | Protein concentrat ion (mg/mL) | Specific activity (U/mg) | Purific ation factor | Yield (%) |
|---|---|---|---|---|
| Crude enzyme | 3.3 | 0.89 | 1.00 | 100 |
| Diafiltratio n | 2.0 | 2.28 | 2.56 | 130 |
| Ion exchange | 1.4 | 3.61 | 4.06 | 54 |
| Concentration | 5.8 | 6.01 | 6.75 | 66 |
| Gel filtration | 0.8 | 10.87 | 12.21 | 64 |
| Concentration | 6.4 | 9.65 | 10.84 | 32 |

The purified LAMAN is stored at -80 °C.

I addition, a purification process for rhLAMAN in 20-200 ml scale is developed for scale-up to large scale production. The quality and purity of the final product (rhLAMAN) is very high and according to the specifications (approved for clinical trials). The process includes a capture step, 1-2 intermediate purification steps, 1 polishing step, 1-2 virus removal steps and 1 formulation step. 1 or more buffer exchange steps are also included (diafiltration). The small-scale process is transferred to intermediate and finally large-scale production.

Experimental design: Several different chromatography gels are tested and performance of the different steps will be analysed by a battery of analytical methods described briefly below:

| | |
|---|---|
| Enzyme activity: | Alpha-mannosidase assay |
| Total protein concentration: | BCA analysis |
| rhLAMAN concentration: | rhLAMAN ELISA |
| Purity: | HPLC and SDS-PAGE |
| Identity: | HPLC |
| HCP proteins: | ELISA |
| Endotoxin level: | Outsource to contract Lab |

### Outline of purification process in 20 ml column scale

### Step 1: Concentration/Diafiltration

Media produced in T-500 flasks (0.06U/ml) with expressed rhLAMAN is concentrated approximately 10x for using Tangential flow filtration (TFF) against a Pellicon Biomax polysulphone filter with 100 kDa cut off.

Example: 0.5 - 1.0 litre spinner produced media is concentrated using TFF against a 50 cm² Biomax 100 kDa filter to 50 - 100 ml. Transmembrane pressure (TMP) is 25 (Pin = 30 psi; Pout = 20 psi).
After that, diafiltration against 7 volumes of 20 mM Tris-HCl, pH 7.6 with TMP of 25 is applied. Specific activity of concentrated sample is 0.5 - 1.5 U/mg. Yield 70 - 90%

### Step 2: Capture step - DEAE sepharose FF

Concentrated sample from step 1 is applied on a 20 ml DEAE sepharose packed in a 16 mm diameter column (Pharmacia XK 16) equilibrated with 20 mM Tris-HCl pH 7.6 (referred to as: standard buffer). Flow rate is 3 ml/min. Protein bound to the DEAE gel is then washed with 2-4 column volumes (CV) of standard buffer followed by 2-4 CV's of 30 mM NaCl in standard buffer.

rhLAMAN is eluted with a linear gradient from 30 - 300 mM NaCl in standard buffer for 20 minutes. Alternatively for the large scale production: Elution is achieved by applying 75 - 150 mM NaCl in standard buffer. The DEAE gel is washed with 2-4 CV's of 0.5 M NaCl in standard buffer followed by 2.4 CV's of 1.0 M NaOH (contact time 40 - 60 minutes). Fractions containing rhLAMAN activity are pooled (specific activity: 2 - 5 U/mg) and used for further purification. Yield 70 - 90 %.

### Step 3: Intermediate step 1

1. *Hydrophobic interaction chromatography: butyl, phenyl or octyl sepharose FF.* Sample pool from step 2 is mixed 1:1 with 2.0 M Na₂SO₄ and applied on a 20 ml HIC column (butyl-, phenyl- or octyl-sepharose) packed in a 16 mm diameter column (Pharmacia XK 16) equilibrated with standard buffer + 1.0 M Na2SO4. Flow rate is 3-4 ml/min. Column is washed with 2-4 CV's of the same buffer. rhLAMAN is eluted with standard buffer and fractions containing activity are pooled and used for further purification.
2. *Macroprep, Ceramic hydroxyapatite type I or II.*
   *Brief description:* Equlibrate column (Gel volume = 20 ml) with 4-6 CV's of 10 mM Sodium phosphate H 7.6 (Buffer A). Flow rate 2-5 ml/min. Buffer exchange sample from step 3 with TFF to Buffer A. Load sample from step 3 on column. Wash with 2-4 CV's of Buffer A. Elute with buffer A containing 100 mM NaCl. Collect peak containing rhLAMAN activity.

### Step 4: Intermediate step 2

1. *Macro prep, Ceramic hydroxyapatite type I or II, 40 um.*
   *Brief description:* see Step 3
2. *Source 15 Q - anioexchanger*
   Equilibrate column with 4 CV's of 200 mM Tris-HCl pH 7.6. Change to 5 CV's of 20 mM Tris-HCl pH 7.6 (standard buffer). Flow rate: 2- 5 ml/min. Load sample from step 3 (should be in standard buffer before application) onto column. Wash with 2-4 CV's of standard buffer. Apply a shallow gradient from 0 to 100% of 1 M NaCl in standard buffer (flow rate 2ml/min, gradient time 50 minutes). Collect fractions containing rhLAMAN activity.
*3. Source 15 S - cation exchanger*
   Should run in acidic pH. Equilibration buffer = 20 mM Sodium Acetate pH 4.5. Elute with a NaCl (increasing salt concentration) or pH (increasing pH) gradient.

### Step 5: Polishing step

1. *Macro prep, Ceramic hydroxyapatite type II, 40 um.*
   Description of parameters: see Step 3.
2. *Source 15 Q - anion exchanger*
   Description of parameters: see Step 4.
3. *Source 15 S - cation exchanger*
   Description of parameters: see Step 4.
*4. Affinity chromatography*

### Step 6: Diafiltration / Formulation step

Tangential flow filtration (TFF) against a Pellicon Biomax polysulphone filter with 100 kDa cut off against 5-10 x Volumes of formulation buffer is performed. Two formulation buffers are tested:
Formulation buffer 1.

| | |
|---|---|
| Na₂HPO₄ | 3.10-3.50 mM |
| NaH₂PO₄ | 0.4-0.6 mM |
| Glycine | 25-30 mM |
| Mannitol | 220-250 mM |

Water for injection (WFI)
Formulation buffer 2.

| | |
|---|---|
| Sodium Citrate | 4.0-5.0 mM |
| Citric Acid | 0.3-0.8 mM |
| Mannitol | 200-250 mM |
| Tween 80 | 3.0-5.0 mM |

Water for injection (WFI)

The pH and osmolality in both Formulation buffers are balanced to 7.5 ± 0.2 and 300 ± 50 mOsm/kg respectively. Final protein concentration is according to the specification ( >5 mg/ml).

### Step 7: Formulation, Filling and Freeze-drying

### Formulation and dosage form

In the development of dosage form the stability of rhLAMAN is focused. The development process starts with an aqueous solution and will, most likely, end up as a freeze-dried product. Two different formulations are tested: Formulation buffer 1 and Formulation buffer 6, see Step 6.

Both these formulations are known to stabilize proteins in aqueous solutions as well as in freeze-dried powders. The pH and osmolality in both Formulation buffers will be balanced to 7.5 ± 0.2 and 300 ± 50 mOsm/kg respectively. Final protein concentration should be according to the specification and in the range 4-10 mg/ml.

A freeze-dried product of rhLAMAN will be produced at a production unit according to EU GMP practice. The filling and freeze-drying will be performed in a room classified as Class A. During production the filling zone is monitored with particle count and settle plates. The personnel are regularly trained according to EU GMP and monitored after each production with glove prints. The sterility of equipment and materials are secured by validated sterilization procedures.

### Filling

The bulk drug substance of rhLAMAN are aseptically filled in sterile type I glass vials.

### Freeze-drying

The vials are freeze-dried with freeze-drying cycles specifically developed for rhLAMAN in the two different formulations described above. Nitrogen gas is filled into the vials in the end of the cycle and eventually closed with stoppers and capped. The batch is finally analyzed and released according to the specification.

### Determination of phosphorylation of LAMAN

LAMAN was incubated overnight with a MPR 300 affinity matrix as described (20). LAMAN activity was determined in the unbound fraction and the fractions eluted with 5 mM glucose 6-phosphate and 5 mM mannose 6-phosphate.

### Results

LAMAN purified from three different species was used for enzyme replacement in α-mannosidosis mice (Fig. 1). The LAMAN from bovine kidney is represented by a mixture of polypeptides (11-38 kDa) generated by limited proteolysis from a common precursor (19). As expected for a lysosomal hydrolase purified from tissue and thereby having been exposed to endosomal/ lysosomal phosphatase activity, the enzyme has a low content of Man6P-recognition marker. When incubated with a Man6P-receptor affinity matrix, 6.4% of the LAMAN activity bound to the matrix in a Man6P-dependent manner.

The recombinant mouse and human LAMAN were isolated from the secretions of Man6Preceptor deficient mouse fibroblasts and of CHO cells, respectively. The enzymes were largely represented by their precursor forms, but contained a variable fraction (5-35%) of proteolytically processed forms of 55 and 70 kDa (Fig. 1). The mouse LAMAN had a higher content of Man6P-recognition marker, which mediated binding of 73.6% of the activity to the affinity matrix. Of the human LAMAN only 4.2% bound in a Man6P-dependent manner to the Man6P-receptor affinity matrix, indicating that the content of Man6P-recognition marker is as low as for the bovine LAMAN.

Several batches have subsequently been analysed, for example:

| | |
|---|---|
| pooled batch 2003-09 - 2004-02: | 40 % phosphorylation |
| batch 2004-02-02 - 06: | 30 % phosphorylation |
| pooled batch 2003-01-21 - 04-16 | 42 % phosphorylation |

In general approximately 40% of the amount of enzyme binds to the affinity matrix. It is believed that the low degree of phosphorylation observed for the human and bovine LAMAN results from prolonged storage of the enzymes.

### Example 2: Effects of the in vivo administration of LAMAN on the levels of stored oligosaccharides

### Experimental procedures

### Injection of the mice

LAMAN was injected into the tail vein of 8-14 week old α-mannosidosis mice (final volume up to 5,3µl/g body weight). Mock-injected mice received the same volume of 10 mM phosphate, pH 7.4 in 0.15 M NaCl (PBS). In a single experiment the mice originating from up to three litters did not differ in age. 5 min after injection, blood was taken from the retroorbital plexus to control for the amount of injected enzyme. Serum was prepared and stored at -20°C.

### Preparation of organ extracts

Mice were anaesthetized with 20 µl of a solution of 10 mg/ml Ketavet (Parke Davis) and 2 mg/ml Rompun (Bayer) in 0.15 M NaCl and perfused with PBS. Organs (liver, spleen, kidney, heart and brain) was collected and stored at -78°C. About 50-70 mg of each organ were homogenized at 4°C in 9 volumes (per weight) of 10 mM Tris/HCl, 150 mM NaCl, 1 mM PMSF (in isopropanol), 1 mM iodoacetamide, 5 mM EDTA. Triton X-100 was added to a final concentration of 0.5% w/v (1% for liver). After incubation for 30 minutes on ice, the samples were sonicated and then centrifuged for 15 minutes at 13000 g. The supernatant was stored at -20°C.

### Enzyme assays

For determination of LAMAN activity in the organ extracts and in serum, 10-50 µl of enzyme sample, was incubated in 0.2 ml of 0.2 M sodium citrate pH 4.6, 0.08% NaN₃, 0.4% BSA, 0.15% NaCl, and 10 mM p-nitrophenyl-α-mannopyranoside as substrate for 0.5-5h at 37°C. 1 ml of 0.4 M glycine/NaOH, pH10.4 was added to stop the reaction. Absorbance was read at 405 nm (ε = 18500 M⁻¹cm⁻¹). All the determinations were done in duplicate and with the appropriate blanks.

### Western blotting

For Western blotting of human LAMAN 20-40 µg of protein was separated on a 10% SDS-polyacrylamide gel. After SDS-PAGE electrophoresis, the proteins were transferred to PVDF membranes using a semi-dry blotting system. Transfer efficiency was checked with Ponceau staining. The membranes were subsequently blocked with 5% skimmed milk powder and incubated with the primary antibody in a 1:50.000 or 1:100.000 dilution. After washing in PBS, 0.1% Tween 20, the blots were incubated with horseradish peroxidase (HRP) coupled secondary antibodies (1:20.000). Signals were visualised using the ECL-Detection System (Amersham, Freiburg, Germany).

### Isolation of neutral oligosaccharides

Tissue samples (50-60 mg) were cut into small pieces and homogenized with 0.6 ml H₂O (HPLC grade) at 4°C. After freezing (-20°C) and thawing twice and ultrasonic treatment, proteins were precipitated by the addition of 4 vol methanol and extracted by the addition of 4 vol chloroform/H₂O (1:3) (21). The supernatant was desalted by incubation for 1 h at 4°C with mixed-bed ion-exchange resin (AG 501-X8, 20-50 mesh). The unbound material was lyophilized and resuspended in water (1 ml per mg tissue).

### Separation of mannose oligosaccharides by thin layer chromatography (TLC)

Neutral oligosaccharides extracted from equal aliquots of tissue were loaded onto TLC plates (20x20 Silica gel F60, Merck). After drying the plates at room temperature for ∼ 1h, the oligosaccharides were separated by developing overnight with n-butanol / acetic acid / H₂0 (100:50:50). After drying (∼ 1h at room temperature and then 5 min at 110°C), the plates were developed for 4 h in n-propanol / nitromethan / H₂0 (100:80:60). For staining, the plates were sprayed with 0,2% orcinol in H₂SO₄ (20% in water), and heated at 110°C. The size of the oligosaccharides was determined by MALDI-TOF (see below).

### Digestion of liver oligosaccharides with α-glucosidase or jack bean α-mannosidase

To avoid the interference with glycogen derived oligosaccharides 20 µl of the oligosaccharide extracts from liver and heart were incubated overnight at 37°C with 40 U/ml α-glucosidase from *Bacillus stearothermophilus* (Sigma) in 20 mM phosphate, pH 6.8. The incubation mixture was heated at 96°C to denature the proteins. After centrifugation 10 min at 13000 g, the supernatant was desalted by incubation 1h at 4°C with a ion-exchange resin (AG 501-X8, 20-50 mesh), lyophilized and resuspended in 20 µl of water, and then separated by TLC.

To verify the nature of the oligosaccharides, 20 µl of the oligosaccharide extracts were incubated overnight at 37°C with 30 U/ml α-mannosidase from Jack bean (Sigma) in 0.1 M sodium acetate, pH 5.0, containing 2 mM ZnCl₂. After incubation the oligosaccharides were prepared as described for samples digested with α-glucosidase and separated by TLC.

### Quantitative analysis of neutral oligosaccharides in organs

The oligosaccharides (0.3 µl) were mixed with 220 pmol of a decasaccharide which served as an internal standard and had the composition GlcNAc₄Man₃Gal₃. The mixture was lyophilized and resuspended in 5 µl of a DMSO / acetic acid (7:3) containing 0.34 M 2-anthranilamide (Aldrich) and 1 M NaBH₃CN (Fluka). After incubation 2 h at 65°C, the samples were purified by paper chromatography (developed with ethyl acetate). The oligosaccharides, localized at the starting point, were extracted by sonicating the paper in water, lyophilized, resuspended in 300 µl acetonitrile / 80 mM ammonium formiate pH 4.4 (65:35), loaded onto a Gluco-Sepharose column (Ludger) and eluted at a flow rate of 0.4 ml/min with the acetonitrile / ammonium formiate buffer. Fluorescence (excitation 350 nm, emission 450 nm) was recorded (Shimadzu, RF-10A XL) and the mass of the oligosaccharides determined by MALDI-TOF.

### MALDI-TOF

Samples from HPLC fractions were were lyophilised and dissolved in 2-3 µl water. Water extracts from TLC plates were derivatized with 1-phenyl-3-methyl-5-pyrazolone and dissolved in 2-3 µl water. 2,5-dihydroxybenzoic acid (DHB, 5 mg/ml in water) was used as matrix. 0.5 µl of DHB and 1 µl of sample were spotted onto the Anchorchip target (Bruker Daltonik), and dried under room temperature. Mass spectrometric analysis was performed on a Reflex III MALDI-TOF (Bruker Daltonik) with a 337nm UV laser.

### Histological examinations

For electron microscopy, small sections of liver and kidney collected at killing time were immersed in 0.1 M phosphate, 6% glutardialdehyde pH 7.4. Tissue samples were post-fixed with 2% osmium tetroxide, dehydrated and embedded in Araldite. Semi-thin sections were stained with toluidine blue. Ultrathin sections were processed according to standard techniques. For histochemical investigations, the sections were inmersed with Bouin's solution diluted 1:4 in 10 mM phosphate pH 7.4, 0.15 M NaCl. Embedding was performed in low melting point paraffin (Wolff, Wetzlar, Germany). Serial sections (7 µm) were cut and mounted on glass slides covered with Biobond (British Biocell, London, UK). Central sections of each series were stained with haematoxylin and eosin for standard light microscopy.

### Results

### Corrective effect of a single intravenous injection of mouse LAMAN

In order to study the short and the long term effect of a single dose of LAMAN on the storage of neutral oligosaccharides, α-mannosidosis mice at the age of 9 weeks received 100 mU mouse LAMAN per g body weight intravenously. To control for the amount of injected enzyme and its clearance from circulation blood was taken 5, 30 and 60 min after injection. 5 min after injection the LAMAN activity varied by less than ± 6% indicating that the mice had received comparable amounts of enzyme. The enzyme was cleared from circulation with a half life of less than 20 min. The mice were killed 2, 4 and 10 h after injection and organ extracts were prepared for determination of LAMAN activity and neutral oligosaccharides. The maximum activity of LAMAN in the organs was observed 2-4 h after injection (Table 1). In liver the activity was 6-7 times higher than in control mice, but also in spleen and heart the maximum values exceeded those of controls. In kidney at maximum one fourth of the activity in control mice was reached. The small activity seen in brain is likely to be LAMAN present in the vascular system. Between 4 and 10 h after injection the enzyme activity decreased rapidly in liver, spleen and kidney with an apparent half life of about 3h. Western blot demonstrated that the internalised precursor of LAMAN was rapidly processed to mature forms (not shown).

The hallmark of α-mannosidosis is the storage of neutral oligosaccharides in a wide variety of tissues (see Fig. 2, lane 1 and lane 2). These oligosaccharides contain 2-9 mannose residues and an N-acetylglucosamine residue at their reducing terminus and therefore result from the action of an endoglucosaminidase. The amount of neutral oligosaccharides in liver and spleen decreased progressively with time to 15% and to 7% of that in mock-injected animals, while in kidney and heart the storage was reduced only to about 49% and 74% (Fig. 2).

**TABLE 1: LAMAN activity in tissue extracts of control and α-mannosidosis mice before and after injection of 100 mU mouse LAMAN per g body weight. * +/+ refers to control mice, - /- to α-mannosidosis mice, and n to the number of animals investigated.⁺ All values were corrected for the mean α-mannosidase activity in serum at t_{zero} (2350 mU/ml serum). The correction factors were 0,98, 1,07 and 0,96 for 2, 4 and 10h respectively.**

| Genotype | **LAMAN injected (100mU/g body weight)** | **LAMAN (mU/g wet weight)** | | | | |
|---|---|---|---|---|---|---|
| | | ***Liver*** | **Spleen** | **Kidney** | **Heart** | **Brain** |
| (+/+)* (n) | - | 136,7±29,1 (19) | 116,3±44,5 (3) | 168,4±34,5 (9) | 12,6±3,1 (3) | 25±1 (2) |
| (-/-)* (n) | - | 2,9±2,5 (21) | 2,8±2,1 (4) | 2,5±1,1 (10) | 0,6±0,5 (3) | 0,4±0,3 (3) |
| | 2h⁺ | 1057 | 185 | 26 | 22 | 2,9 |
| | 4h⁺ | 883 | 183 | 40 | 5 | 1,4 |
| | 10h⁺ | 197 | 36 | 10 | 2 | 1,0 |

To determine how long the corrective effect of a single dose of LAMAN persists, mice were examined 4, 8 and 16 days after the injection of 100 mU mouse LAMAN per g body weight. At all time points the LAMAN activity in the organs (spleen, kidney, heart, brain) was in the range of non-treated or mock-injected α-mannosidosis mice, except for liver, where 4 days after injection the LAMAN activity (15.1 mU/g wet weight) was still about 5-fold higher than in mock-injected mice. The storage of neutral oligosaccharides in liver, spleen and kidney 4 days after injection was in the range seen 10 h after injection (compare Fig. 2 and Fig. 3). In heart storage had decreased from 74% after 10 h to 42% after 4 days. This indicates that the corrective effect seen after 10 h persists for about 4 days in spite of the fact that little or no LAMAN activity is detectable 10 h after injection in organs such as kidney or heart. After 4 days the storage of oligosaccharides clearly began to increase again. The increase observed between day 4 and day 16 after injection corresponded to 20-40% of the storage seen in mock-injected α-mannosidosis mice (Fig. 3).

### Comparison of the clearance and corrective effect of bovine, mouse and human LAMAN

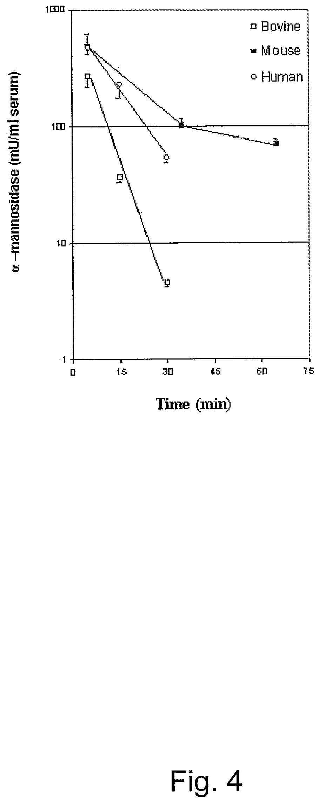

To compare the corrective effect of the LAMAN preparations from the three sources mice were injected with a dose of LAMAN that was expected to yield a partial correction. We therefore injected 50 mU LAMAN per g body weight. The bovine and the human LAMAN were rapidly cleared from circulation with half lifes of 4 min and 8 min, respectively (Fig. 4). The clearance of the highly phosphorylated mouse LAMAN was slower and at least biphasic. About 85% of the enzyme were cleared with an apparent half time of 12 min, while the apparent clearance of the remaining fraction was about 47 min (Fig. 4).

The mice were killed 2 days after injection and extracts from liver, spleen, kidney and heart were examined for neutral oligosaccharides (Fig. 5). With the exception of liver the corrective effect was highest for the human LAMAN and lowest for the bovine LAMAN. The corrective effect of the mouse enzyme was intermediate. Only in liver was the corrective effect of the bovine enzyme (4% of the storage remaining) more pronounced than that of the human enzyme (14% of the storage remaining). In liver the corrective effect of mouse LAMAN was weakest (23% of the storage remaining).

### Corrective effect of human LAMAN at high dose

The comparison of the bovine, mouse and human LAMAN indicated that the poorly phosphorylated human LAMAN had a relatively higher corrective potential in kidney and heart, two organs which are more resistant to metabolic correction than liver and spleen. To evaluate the corrective potential of the human enzyme we injected a single dose of 250 mU LAMAN and analysed the mice 1 to 12 days after injection. In liver storage was fully corrected 1 and 3 days after injection. After 6 and 12 days neutral oligosaccharides started to accumulate again, but reached only about 30% of the storage level before treatment (Fig. 6). Light microscopical examination of the liver revealed the almost complete disappearance of storage vacuoles, which are prominent in sinus endothelial cells, Kupffer cells and hepatocytes of untreated α-mannosidosis mice and reappear 12 days after injection (Fig. 7). In spleen and kidney, the storage of neutral oligosaccharides decreased to 12 and 18%, respectively. It is noteworthy, that in spleen and kidney the maximum correction was observed after 3 and 6 days, respectively. In both organs neutral oligosaccharides started to reaccumulate 3 and 6 days after the injection (see Fig. 5). The neutral oligosaccharides in the brain of α-mannosidosis mice were not affected by the treatment (not shown).

The human LAMAN activities recovered 24 h after injection were much higher than expected from the experiments with mouse LAMAN. In liver the human LAMAN activity was still 6 times higher than in control liver. In spleen and kidney it accounted for 10-15% of that in control. To follow the uptake and stability of human enzyme, LAMAN activity was determined in tissue extracts prepared 4, 16 and 24 h after injection of 250 mU human LAMAN per g body weight. Less than 20% of the injected LAMAN was recovered after 4 h in the tissues examined (liver 18%, kidney 0.4%, spleen 0,12% and heart 0,04%). When compared to the uptake of mouse LAMAN and taking into account that a 2.5 fold higher amount of human LAMAN was injected, the activity recovered in liver, kidney and heart 4 h after injection was comparable for both enzyme preparations (compare Table 1 and Table 2). Uptake by spleen appeared to be 2-3 times less efficient for the human enzyme. The biggest difference between the human and mouse enzyme was the higher stability of the human LAMAN internalized by liver, kidney and spleen. While the activity of the human LAMAN had decreased in these organs after 24 h to 17-33% of the 4 h value (see Table 2), that of the mouse enzyme had decreased to 20-26% of the 4 h value already after 10 h (see Table 1).

The corrective effect of the mouse (see Fig. 3) and human LAMAN was only transient. Neutral oligosaccharides started to reaccumulate 3 to 6 days after injection. Increasing the amount of injected enzyme would be a means to delay the reaccumulation. The corrective effect of a given dose of LAMAN is expected to be higher when administered as two half doses separated by an appropriate interval than as a single dose. Rather than increasing the amount of LAMAN to 500 mU/g body weight we administered two times 250 mU human LAMAN per g body weight each at day 7 and 3.5 prior to the analysis. This resulted in a full correction of the storage in kidney and heart (Fig. 8). Light microscopical examination demonstrated the absence of storage vacuoles in liver (not shown) and in tubular epithelia of kidney (Fig. 9). The residual storage in spleen was less than 20% of that in mock-injected α-mannosidosis mice. Most notably, in brain the level of neutral oligosaccharides as quantified by TLC was only half of that in the brain of mock-injected α-mannosidosis mice (Fig. 8).

**TABLE 2 : LAMAN activity in tissue extracts of α-mannosidosis mice 4-24 h after injection of 250 mU of human α-mannosidosis per g body weight.⁺ All values were corrected for the mean LAMAN activity in serum at t_{zero}(5012 mU/ml serum).The correction factors were varied between 0,903 and 1,222.* For LAMAN activity in tissue extracts of control mice and non-injected mice see Table1.**

| **Hours after injection** | **LAMAN (mU/g wet weight)*** | | | | |
|---|---|---|---|---|---|
| | ***Liver*** | **Spleen** | **Kidney** | **Heart** | **Brain** |
| 4 | 2774 | 157 | 81 | 21 | 2,1 |
| 8 | 2639 | 65 | 45 | 8 | 1,7 |
| 16 | 1549 | 31 | 26 | 10 | 2,7 |
| 24 | 796 | 24 | 16 | 6 | 3,0 |

To quantify the neutral oligosaccharides on a molar basis the neutral oligosaccharides extracted from the brain of the mock- and LAMAN-injected α-mannosidosis mice were reacted with 2-anthranilamide, which introduces a fluorescent tag at the reducing termini of the oligosaccharides. The fluorescently labelled oligosaccharides were separated by HPLC and quantified by comparison with an internal standard oligosaccharide added prior to the derivatization with 2-anthranilamide (Fig. 10). The major oligosaccharide species in brain of α-mannosidosis mice contain 2, 3 or 4 mannose residues. They are present at concentrations of 319, 87 and 164 pmol/g tissue, respectively, and account for 61% of all neutral oligosaccharides in brain. In the brain of treated α-mannosidosis mice the concentration of the neutral oligosaccharides decreased to 26% of mock-injected mice. The corrective effect was noted for each of the oligosaccharide species but was relatively highest for the species with 9, 7 and 4 mannose residues (see Fig. 10).

In the present study LAMAN purified from bovine kidney was used. This enzyme preparation consisted of mature polypeptides with a low mannose 6-phosphate content. The recombinant human LAMAN isolated from the secretion of CHO cells consisted mainly of the precursor form, but was also poorly phosphorylated. The low mannose 6-phosphate content of this preparation is likely to be due to dephosphorylation during isolation. Occasionally, human LAMAN preparations were obtained of which up to 40% bound to the MRP 300 column (C. Andersson, D. P. Roces, unpublished observation). The recombinant mouse LAMAN purfied from the secretions of mouse embryonic fibroblasts that lack mannose 6-phosphate receptors, was largely phosphorylated and represented by the precursor form. The clearance from circulation was faster for the two poorly phosphorylated LAMAN preparations from bovine kidney and CHO cells than for the highly phosphorylated mouse LAMAN. The uptake of the different LAMAN preparations by organs was not directly compared. However, comparing the data of different experiments suggests that of the poorly phosphorylated human and of the highly phosphorylated mouse LAMAN similar fractions are taken up by the liver, kidney and heart. Only in spleen was the uptake different and facilitated by higher phosphorylation. In a related study on the uptake of phosphorylated and non-phosphorylated β-glucuronidase in mice no difference was observed for the uptake into liver and spleen, while uptake into kidney and heart was higher for the phosphorylated enzyme (26). For α-galactosidase preparations that differed in glycosylation and phosphorylation a similar uptake by liver, spleen and kidney was observed (27). This indicates that the effect of glycosylation and phosphorylation on the uptake of lysosomal enzymes into different organs depends on the enzyme.

A major difference between the LAMAN preparations from different sources are their stability. Assuming first order kinetics the activity of mouse (see Table 1) and bovine (not shown) LAMAN decreased in liver, kidney and spleen with an apparent half life of 3 h or less, while the apparent half life of human LAMAN was more than 3 times longer. This may explain the higher corrective efficacy of human LAMAN on the oligosaccharide storage in spleen, kidney and heart (see Fig. 5).

A single intravenous administration of LAMAN led to a rapid and pronounced decrease of the lysosomal storage of neutral oligosaccharides irrespective of the source of the enzyme. The corrective effect was most pronounced in liver. This is also the tissue where the increase of LAMAN activity relative to that in wild type mice was highest. Histological examination revealed a correction of storage in both parenchymal and non-parenchymal liver cells. This was true for the highly phosphorylated mouse LAMAN (not shown) and for the poorly phosphorylated human LAMAN (see Fig. 7). This was unexpected as an earlier study with β-glucuronidase had shown that non-phosphorylated enzyme forms localized almost exclusively to non-parenchymal liver cells, while the phosphorylated forms localized to both parenchymal and non-parenchymal liver cells (26). The corrective effect of LAMAN was transient in all tissues. The neutral oligosaccharides started to reaccumulate 2 to 6 days after the injection.

A remarkable correction of storage was observed when the injection of 250 mU human LAMAN per g body weight, which reduces storage in spleen and kidney by 80-90% was repeated after 3.5 days. Within one week the storage disappeared not only in liver, but also in kidney and heart. More importantly, the concentration of neutral oligosaccharides also decreased in brain to about one fourth. The latter cannot be attributed to an uptake of LAMAN into neural cells. In mice the blood-brain barrier matures within the first two weeks of life, and lysosomal enzymes administered intravenously after this period do not cross the blood-brain barrier (28-31). In accordance with these observations we observed trace amounts of LAMAN in the brain homogenates of treated mice (see Table 1 and 2). These activities are attributed to extraneural cells, such as endothelia, choroid plexus epithelium and incompletely removed meninges. As it is unlikely that the administered LAMAN gets access to oligosaccharides stored in neuronal and glial cells alternative mechanisms have to be considered. Clearance of oligosaccharides via the blood circulation or via an improved flow of the cerebrospinal fluid may contribute to the decrease of neutral oligosaccharide storage in brain.

### Example 3: rhLAMAN - Efficacy of repeated dose replacement therapy in α-mannosidosis mice

The study objective is to
- Demonstrate a total correction of the mannose oligosaccharides storage in all the peripheral tissues, and verify the correction of the storage in the brain.
- Determine if the injection of a repeated dose of rhLAMAN has also an effect on the LAMAN activities measured in the different peripheral tissues.
- Follow the development of antibodies after the repeated injections, and check whether they precipitate the enzyme. Determine if, once the mice developed antibodies, these have an effect on the clearance of the enzyme.
- Determine whether an increased level of antibodies has an effect on the permeability of the brain blood barrier (BBB).
- Compare the gene expression of knock-out, injected knock-out and wild type mice after 1 and 4 weeks of treatment. A microarray experiment will be performed with RNA from liver. The mice used for this experiment are selected from heterozygote breedings (see below).

### Justification of test system

### Test item

The test item is supplied as an 5.4 mg.ml⁻¹ solution (8.2 U/mg, batch 041202) and is used as supplied. The test item is stored frozen (ca -20°C) in the dispensary when not in use.

### Animals and management

For the experiment 5 different groups of mice are used:

| | |
|---|---|
| (-/-) | α-mannosidosis mice |
| (+/+) | C57/B6 (wild type mice) |
| (+/-) | heterozygote mice that are offspring of a cross between α-mannosidosis mice (-/-) and C57/B6 mice(+/-) |
| (-/-) | α-mannosidosis mice from heterozygote crosses (+/- mice) |
| (+/+) | wild type mice from heterozygote crosses (+/- mice) |

| | |
|---|---|
| Age: | 8 weeks at initiation of the study. |
| Number of Animals: | 64 (males and/or females) mice |
| Health Status: | Animals suspected of being diseased are culled from the study. If significant numbers of animals are unsuitable, the entire batch of animals is rejected and a new batch obtained. |
| Acclimatisation: | The animals are allowed to acclimatize to the accommodation for a minimum of 3 days prior to the commencement of dosing. |

Allocation to Dose Groups: All the animals are weighed and the required number of animals is randomly assigned to the 5 dose groups. A weight and/or sex stratification is not necessary, as the dose is weight adjusted, and no sex-specific differences were found so far. Only males are used for the Microarray experiment (groups 2*, 4* and 5*, see below), since in gene expression studies, sex has frequently been described as a relevant factor.

Room Environment: The animals are housed 4/cage or 8/cage in a polypropylene cage with stainless steel mesh tops and solid bottoms with wood shavings as bedding material. Cages (267 x 207 x 104 mm or 425 x 266 x 188 mm) are changed as necessary.

The cages are supplied with a polycarbonate water bottle (capacity 250 ml) with a stainless steel nozzle and Durethane cap. Water bottles are filled as necessary and changed/washed at least once weekly.

There is automatic control of temperature and humidity, target ranges are 20°C± 2°C and 50%±15%, respectively, with a room airflow intended to give a minimum of 15 air changes per hour. Temperature and humidity ranges are monitored and recorded daily. There are automatic control of light cycle; light hours are normally 0600-1800.

### Diet and Water Supply

Food and water are available to the animals ad *libitum.* The food and water used are not considered to contain any additional substances, in sufficient concentration, to have any influence on the outcome of the study.

### Treatment

### Dose Groups/Dose Level

Group 1 (for biochemistry investigations, 16 LAMAN mice). Mice are treated twice a week intravenously with 250 mU/g body weight rhLAMAN, and groups of 4 mice are killed after 1, 2 and 4 weeks of treatment.

Group 2* (for microarray investigations, 8 LAMAN mice from heterozygous cross), are treated twice a week intravenously with 250 mU/g body weight rhLAMAN, and groups of 3 mice are killed after 1 and 4 weeks of treatment.

Group 3 (for blood-brain-barrier, Antibody and plasma clearance investigations, 12 LAMAN mice), are treated once weekly with 250 mU/g body weight rhLAMAN. Both groups of mice, the 4 mice used for BBB investigations, and the 4 mice used for clearance investigation, are killed after 4 weeks of treatment.

Group 4 (Mock treated mice, 12 LAMAN mice) are treated once/twice weekly with vehicle for rhLAMAN at the same dose volume as the treated animals in Group 3/Group 1. A group of 4 mice once a week injected are killed after 4 weeks of treatment (to be compared with mice from group 3), and groups of 2 mice are killed after 1, 2 and 4 weeks of treatment (to be compared with group 1)

Group 4* (Mock treated mice, 8 LAMAN mice from heterozygous cross) and Group 5* (8 wild type from heterozygous cross) are treated twice a week with vehicle for rhLAMAN at the same dose volume as the treated animals in Group 2*. Groups of 3 mice are killed after 1 and 4 weeks.

*α-Mannosidosis mice from heterozygote breedings (+/-), offspring of a breeding between α-mannosidosis mice (-/-) and wild type C57/B6 (+/+).

**TABLE 3: Allocation of animals to dose groups:**

| Animals | | LAMAN knock-out mice (LAMAN injected) | | | | | | | No of Injections |
|---|---|---|---|---|---|---|---|---|---|
| Group No | | 1A | | 1C | 2A* | 2B* | 3A | 3B | |
| Treatment time | Killing times | | | | Microarray | | Evans Blue | Ab-mice | |
| to | | 36x | + | + | + | + | + | + | 36x |
| t_{0,5w} | | 24x | + | + | + | + | | | 24x |
| t_{1w} | t_{1w} | 29x | + | + | 3* | + | + | + | 29x |
| t_{1,5w} | | 17x | + | + | | + | | | 17x |
| t_{2w} | t_{2w} | 25x | 4 | + | | + | + | + | 25x |
| t_{2,5w} | | 13x | | + | | + | | | 13x |
| _{t3w} | | 25x | | + | | + | + | + | 25x |
| t_{3,5w} | | 13x | | + | | + | | | 13x |
| t_{4w} | t_{4w} | - | | 4 | | 3* | 4 | 4 | - |
| | | | | | | | | | |
| No | | | 4 | 4 | 3 | 3 | 4 | 4 | |
| Spar e | | | | +4 | | +2 | | +4 | |

**TABLE 3: Allocation of animals to dose groups (continued)**

| Animals | | LAMAN knock-out mice | | | | | | Wild Type mice | | No of Injections |
|---|---|---|---|---|---|---|---|---|---|---|
| Group No | | 4A | 4B | 4C | 4D | 4E* | 4F* | 5A* | 5B* | |
| Treatment time | Killing times | Control Mock treated | | | | | | Controls Mock Tr. | | |
| to | | + | + | + | + | + | + | + | + | 36x |
| t_{0,5w} | | + | + | + | | + | + | + | + | 24x |
| t_{1w} | t_{1w} | 2 | + | + | + | 3* | + | 3* | + | 29x |
| t_{1,5w} | | | + | + | | | + | | + | 17x |
| t_{2w} | t_{2w} | | 2 | + | + | | + | | + | 25x |
| t_{2,5w} | | | | + | | | + | | + | 13x |
| t_{3w} | | | | + | + | | + | | + | 25x |
| t_{3,5w} | | | | + | | | + | | + | 13x |
| t_{4w} | t_{4w} | | | 2 | 4 | | 3* | | 3* | - |
| | | | | | | | | | | |
| No | | 2 | 2 | 4 | 2 | 3 | 3 | 3 | 3 | |
| Spare | | | | | | | +4 | | +2 | |

### Route, Duration and Method of Administration

The test item is administered via an intravenous (bolus) injection to one of the lateral tail veins at target dose levels. The requisite dose volume is calculated according to the animals most recently recorded body weight.

Prior to the administration, the mice are anesthetized (intra peritoneal) with 75 µl per 100 g body weight of a solution 10 mg/ml Ketavet (Parke Davis) and 2 mg/ml Rompun (Bayer), and are kept by 37 °C until they are recovered from anesthesia.

### Observations

### Viability

All animals are checked early morning and as late as possible each day for viability. Any animal showing signs of severe debility or intoxication and considered to be in a moribund state, is killed. Any animal killed in extremis or found dead are, whenever possible, subjected to a macroscopic post mortem examination on the day of death. If it is not feasible to undertake the macroscopic post mortem examination on the day of death (eg late afternoon or weekends), the carcass is placed in a polythene bag and kept in a refrigerator (4°C) until examination the following day.

### Clinical Signs

All animals are observed on days of dosing for reaction to treatment. The onset, intensity and duration of any signs observed is recorded.

### Body Weight

Prior to dosing all animals are weighted weekly and recorded and tabulated.

### Laboratory investigations

### Treatment verification

Five minutes after injection, blood from the retroorbital plexus is taken for measurement of rhLAMAN plasma levels (according to internal procedure no 2). If an animal did not receive the full dose, the % missing amount is calculated and given to the animal on the same day.

### Kinetics

From 4 mice of group 3, blood from the retroorbital plexus is collected one day before the injection, and also 5, 15, and 30 minutes after each injection, to investigate a possible effect of the Ab on the clearance of the enzyme.

When enzyme is still available at the end of the experiment (4 weeks), the treatment of these mice will continue up to 8 weeks.

### Antibody determination

Before dosing blood from retroorbital plexus is taken from group 2 animals as well as of all other animals at termination in order to measure the antibody titers.

### Terminal studies

### Method of killing

Mice are anaesthetized with 600 µl of a solution 10 mg/ml Ketavet (Parke Davis) and 2 mg/ml Rompun (Bayer) in 0.15M NaCl, perfused with PBS and killed by bleeding.

For all groups except group 3, the killing take place three and a half days after last treatment. For group 3, the mice are killed 1 week after last treatment (see table 3).

### Histopathology

The following organs are collected and fixed with 0.1mM NaPi/6% Gluteraldehyd buffered to pH 7.4: liver, kidney, brain

Small sections are cut with razor blade, and the fixed slides are subject toor electron microscopy analysis.

### Tissue LAMAN concentrations

Homogenates of Liver, Spleen, kidney, heart, brain in TBS/PI (Protease Inhibitors) are measured for LAMAN activity using p-nitrophenyl-α-mannopyranoside as substrate and 0,2M sodium citrate pH 4.6, 0,08% NaN₃, 0,4% BSA as buffer.

Processing of LAMAN is followed by Western Blot (∼1mU is needed).

### Tissue Oligosaccharides

From the organs described in 10.3 a preparation of neutral oligosaccharides extracts are prepared and quantified by densitometry to study the normalization of the storage in the organs by TLC according to procedure no 4 and 5.

Neutral oligosaccharides from liver and heart will be digested with α-glucosidase, to avoid the interference of glucogen.

### Statistical analysis of results

Unless otherwise stated, all statistical tests will be two-sided and performed at the 5% significance level using in-house software.

The following pairwise comparisons are performed:
Vehicle Control Group 4A vs Group 1A
Vehicle Control Group 4B vs Group 1B
Vehicle Control Group 4C vs Group 1C
Vehicle Control Group 4D vs Group 3A
Vehicle Control Group 5A*, 5B* vs Group 4E*, 4F*
Vehicle Control Group 5A*, 5B* vs Group 2A*, 2B*
Vehicle Control Group 4E*, 4F* vs Group 2A*, 2B*

The data are analysed for normality of the distribution using the Shapiro-Wilk test and quantile-quantile-plots. When a normal distribution can be assumed, differences between the groups are tested for using linear models (ANOVA) with or without homoschedasticity assumption. If the data (or log transformed data) are not normally distributed, a Kruskal-Wallis-Test is used. Additional data analysis steps may be undertaken depending on the data.

(* heterozygote breedings, see below)

### REFERENCES

1. Ockerman, P. A. (1967) A generalized storage disorder resembling Hurler's syndrome. Lancet 2, 239-241
2. Hocking, J.D., Jolly, R.D,. and Blatt R.D. (1972) Deficiency of α-mannosidase in Angus cattle. An inherited lysosomal storage disease. Biochem. J. 128, 69-78
3. Burditt, L.J., Chtai, K., Hirani, S., Nugent, P.G., Winchester, B. and Blakmore, W.F. (1980) Biochemical studies on a case of feline mannosidosis. Biochem. J. 189, 467-473
4. Hirsch, C., Blom, D. and Ploegh, H.L. (2003) A role for N-glycanase in the cytosolic turnover of glycoproteins. EMBO J. 22, 1036-1046.
5. Saint-Pol, A., Cordogno, P. and Moore, S.E.H. (1999) Cytosol to lysosome transport of free polymannose-type oligosaccharides. J. Biol. Chem. 274, 13547-13555
6. Thomas, G.H. (2001) Disorders of glycoprotein degradation: α-mannosidosis, β-mannosidosis, fucosidosis and sialidosis in "The Metabolic Bases of Inherited Disease (Scriver, C., Beaudet, A., Sly, W., Valle, D., Childs, B., Kinzler, K., Vogelstein, B., eds.) 8th ed., pp. 3507-3533, McGraw-Hill, New York
7. Berg, T., Riise, H.M., Hanse, G.M., Malm, D., Tranebjarg, L., Tollersrud, O.K. and Nilssen, O. (1999) Spectrum of mutations in α-mannosidosis. Am J. Hum. Genet. 64, 77-88
8. Neufeld, E.F. (2004) Enzyme replacement therapy. In "Lysosomal disorders of the brain" (Platt, F.M., Walkley, S.V., eds.) pp. 327-338, Oxford University Press
9. Dobrenis, K. (2004) Cell-mediated delivery systems. In "Lysosomal disorders of the brain" (Platt, F.M., Walkley, S.V., eds.) pp. 339-380, Oxford University Press
10. Barton, N.W., Brady, R.O., Dambrosia, J.M., Di Bisceeglie, A.M., Doppelt, S.H., Hill, S.C., Mankin, H.J., Murray, G.J., Parker, R.I., Argoff, C.E., Grewal, R.P., Yu, K.-T. et al. (1991) Replacement therapy for inherited enzyme deficiency-macrophage-targeted glucocerebrosidase for Gaucher's disease. N. Engl. J. Med. 324, 1464-1470
11. Will, A., Cooper, A., Hatton, C., Sardhawalla, I.B., Evans, D.I.K. and Stevens, R.F. (1987) Bone marrow transplantation in the treatment of α-mannosidosis. Arch. Dis. Childhood 62, 1044-1049
12. Wall, D.A., Grange, D.K., Goulding, P., Danies, M., Luisiri, A. and Kotagal, S. (1998) Bone marrow transplantation for the treatment of α-mannosidase. J. Pediatr. 133, 282-285
13. Malm, P. (2004) Prologue. The doctor as parent. In "Lysosomal disorders of the brain" (Platt, F.M., Walkley S.U., eds.) pp. XXI-XXVII, Oxford University Press
14. Walkely, S.U., Thuall, M.A., Dobrenis, K., Huang, M., March, .A., Siegel, D.A. and Wurzelmann, S. (1994) Bone marrow transplantation corrects the enzyme defect in neurons of the central nervous system in a lysosomal storage disease. Proc. Natl. Acad. Sci. U.S.A. 91, 2970-2974
15. Stinchi, S., Lüllmann-Rauch, R., Hartmann, D., Coenen, R., Beccari, T., Orlaccio, A., von Figura, K., and Saftig, P. (1999) Targeted disruption of the lysosomal α-mannosidase gene results in mice resembling a mild form of human α-mannosidosis. Hum. Mol. Gen. 8, 1365-1372
16. Beccari, T., Apollini, M.G., Constanzi, E., Stinchi, S., Stirling, J.L., della Fazia, M.A., Servillo, G., Viola, M.P., and Orlacchio, A. (1997). Lysosomal alpha-mannosidases of mouse tissues: characteristics of the isoenzymes, and cloning and expression of a full-length cDNA. Biochem J. 327, 45-49
17. Artelt, P., Morelle, C., Ausmeier, M., Fitzek, M., and Hauser, H. (1988). Vectors for efficient expression in mammalian fibroblastoid, myeloid and lymphoid cells via transfection or infection. Gene 68, 213-219
18. Dittmer, F., Ulbrich, E.J., Hafner, A., Schmahl, W., Meister, T., Pohlmann, R., and von Figura, K. (1999). I-cell disease-like phenotype in mice deficient in mannose 6-phosphate receptors. Transgenic Res. 7, 473-483
19. Tollersrud, O.K., Berg, T., Healy, P., Evjen, G., Ramachandran, U. and Nilssen, O. (1997) Purification of bovine lysosomal α-mannosidase, characterization of its gene and determination of two mutations that cause α-mannosidosis. Eur. J. Biochem. 246, 410-419
20. Pohlmann, R., Wendland, M., Boeker, C. and von Figura, K. (1995) The two mannose 6-phosphate receptors transport distinct complements of lysosomal proteins. J. Biol. Chem. 270, 27311-27318
21. Wessel, D. and Flügge, U.I. (1984). A method for the quantitative recovery of protein in dilute solution in the presence of detergents and lipids. Anal. Biochem. 138, 141-143
22. Kakkis, E.D., Muenzer, J., and Tilller, G.E. (2001) Enzyme replacement therapy in mucopolysaccharidosis. N. Engl. J. Med. 344, 182-188
23. Ghosh, P., Dahms, N.M. and Kornfeld, S. (2003) Mannose 6-phosphate receptors: new twists in the tale. Nature reviews 4, 202-212
24. Pontow, S.E., Kery, V., and Stahl, P.D. (1992) Mannose receptor. Int. Rev. Cytol. 137B, 221-244
25. Ashwell, G. and Harford, J. (1982) Carbohydrate-specific receptors of the liver. Ann. Rev. Biochem. 51, 531-554
26. Sands, M.S., Vogler, C.A., Ohlemiller, K.K., Roberts, M.S., Grubb, J.H., Levy, B. and Sly, W.S. (2001) Biodistribution, kinetics and efficacy of highly phosphorylated and non-phosphorylated β-glucuronidase in the murine model of mucopolysaccharidosis VII. J. Biol. Chem. 276, 41160-43165
27. Ioannu, Y.A., Zeidner, K.M., Gordon, R.E. and Desnick, R.J. (2001) Fabry disease: preclinical studies demonstrated the effectivness of α-galacosidase replacement in enzyme-deficient mice. Am. J. Hum. Genet. 68, 14-25
28. Vogler, C., Levy, B., Galvin, N.J., Thorpe, C., Sands, M.S., Barker, J.E., Barty, J., Birkenmeier, E.H., and Sly, W.S. (1999) Enzyme replacement therapy in murine mucopolysaccharidosis type VII: neuronal and glial response to β-glucuronidase requires early initiation of enzyme replacement therapy. Pediatr. Res. 45, 838-844
29. Yu, W.H., Zhao, K.W., Ryanzantsev, S., Rozengurt, N., and Neufeld, E.F. (2000) Short term enzyme replacement in the murine model of Sanfilippo syndrome type B. Mol. Genet. Metab. 71, 753-780
30. Miranda, S.R., He, X., Simonara, C.M., Gatt, S, Desnick, R.J., and Schuchman, E.H. (2000) Infusion of recombinant human acid sphingomyelinase into Niemann-Pick disease mice leads to visceral, but not neurological correction of the pathophysiology. FASEB J. 14, 1988-1995
31. Du, H., Schiavi, S., Levine, M., Mishra, J., Heur, N., and Grabowski, G.A. (2001) Enzyme therapy for lysosomal acid lipasi deficiency in the mouse. Hum. Mol. Genet. 10, 1639-1648
32. Aebischer P, Goddard M, Signore AP, Timpson RL. 1994. Functional recovery in hemiparkinsonian primates transplanted with polymer-encapsulated PC12 cells. Exp Neurol 126:151-158
33. Aebischer P, Schluep M, Deglon N, Joseph JM, Hirt L, Heyd B, Goddard M, Hammang JP, Zurn AD, Kato AC, Regli F, Baetge EE. 1996. Intrathecal delivery of CNTF using encapsulated genetically modified xenogeneic cells in amyotrophic lateral sclerosis patients. Nat Med 2:696-699
34. Austin J, McAfee D, Armstrong D, O'Rourke M, Shearer L, Bachhawat B. 1964. Abnormal sulphatase activities in two human diseases (metachromatic leucodystrophy and gargoylism). Biochem J 93:15C-17C
35. Barth ML, Fensom A, Harris A. 1995. Identification of seven novel mutations associated with metachromatic leukodystrophy. Hum Mutat 6(2):170-176
36. Bayever E, Ladisch S, Philippart M, Brill N, Nuwer M, Sparkes RS, Feig SA. 1985. Bone-marrow transplantation for metachromatic leucodystrophy. Lancet 2(8453):471-473
37. Bradley RT, Manowitz P. 1988. Electrochemical determination of lysosomal alpha-mannosidasectivity using high-performance liquid chromatography. Anal Biochem 173(1):33-38
38. Braulke T, Hille A, Huttner WB, Hasilik A, von Figura K. 1987. Sulfated oligosaccharides in human lysosomal enzymes. Biochem Biophys Res Commun 143(1):178-185
39. Braulke T, Tippmer S, Chao HJ, von Figura K. 1990. Insulin-like growth factors I and II stimulate endocytosis but do not affect sorting of lysosomal enzymes in human fibroblasts. J Biol Chem 265(12):6650-6655
40. Deglon N, Heyd B, Tan SA, Joseph JM, Zurn AD, Aebischer P. 1996. Central nervous system delivery of recombinant ciliary neurotrophic factor by polymer encapsulated differentiated C2C12 myoblasts. Hum Gene Ther 7(17):2135-2146
41. Dierks T, Schmidt B, von Figura K.1997. Conversion of cysteine to formylglycine: a protein modification in the endoplasmic reticulum. Proc Natl Acad Sci USA 94(22):11963-11968
42. Draghia R, Letourneur F, Drugan C, Manicom J, Blanchot C, Kahn A, Poenaru L, Caillaud C. 1997. Metachromatic leukodystrophy: identification of the first deletion in exon 1 and of nine novel point mutations in the lysosomal alpha-mannosidase gene. Hum Mutat 9(3):234-242
43. Draper RK, Fiskum GM, Edmond J. 1976. Purification, molecular weight, amino acid, and subunit composition of lysosomal alpha-mannosidase from human liver. Arch Biochem Biophys 177(2): 525-538
44. Dubois G, Turpin JC, Baumann N. 1975. Arylsulfatases isoenzymes in metachromatic leucodystrophy/detection of a new variant by electrophoresis improvement of quantitative assay. Biomedicine 23(3):116-119
45. Eto Y, Tokoro T, Liebaers I, Vamos E. 1982. Biochemical characterization of neonatal multiple sulfatase deficient (MSD) disorder cultured skin fibroblasts. Biochem Biophys Res Commun 106(2):429-34
46. Farooqui AA. 1976. Purification and properties of human placenta arylsulphatase A. Arch Int Physiol Biochim 84(3):479-492
47. Farrell DF, MacMartin MP, Clark AF. 1979. Multiple molecular forms of lysosomal alpha-mannosidase in different forms of metachromatic leukodystrophy (alpha-mannosidosis). Neurology 29(1):16-20
48. Fujii T, Kobayashi T, Honke K, Gasa S, Ishikawa M, Shimizu T, Makita A. 1992. Proteolytic processing of human lysosomal lysosomal alpha-mannosidase. Biochim Biophys Acta 1122(1):93-98
49. Gieselmann V, Polten A, Kreysing J, Kappler J, Fluharty A, von Figura K. 1991. Molecular genetics of metachromatic leukodystrophy. Dev Neurosci 13:222-227
50. Gieselmann V, Zlotogora J, Harris A, Wenger DA, Morris CP. 1994. Molecular genetics of metachromatic leukodystrophy. Hum Mutat 4:233-42
51. Gieselmann V, Matzner U, Hess B, Lullmann-Rauch R, Coenen R, Hartmann D, D'Hooge R, DeDeyn P, Nagels G. 1998. Metachromatic leukodystrophy: molecular genetics and an animal model. J Inherit Metab Dis 21:564-574
52. Gniot-Szulzycka J. 1974. Some properties of highly purified arylsulphatase A from human placenta. Acta Biochim Pol 21(3):247-254
53. Gustavson KH, Hagberg B. 1971. The incidence and genetics of metachromatic leucodystrophy in northern Sweden. Acta Paediatr Scand 60(5):585-590
54. Hess B, Saftig P, Hartmann D, Coenen R, Lullmann-Rauch R, Goebel HH, Evers M, von Figura K, D'Hooge R, Nagels G, De Deyn P, Peters C, Gieselmann V. 1996. Phenotype of lysosomal alpha-mannosidase-deficient mice: relationship to human metachromatic leukodystrophy. Proc Natl Acad Sci USA 93(25):14821-14826
55. Hickey WF, Kimura H. 1988. Perivascular microglial cells of the CNS are bone marrow-derived and present antigen in vivo. Science 239(4837):290-2
56. Hickey WF, Hsu BL, Kimura H. 1991. T-lymphocyte entry into the central nervous system. J Neurosci Res 28(2):254-60
57. Inoue H, Seyama Y, Yamashita S. 1986. Specific determination of lysosomal alpha-mannosidase activity. Experientia 15;42(1):33-35
58. James GT. 1979. Essential arginine residues in human liver lysosomal alpha-mannosidase. Arch Biochem Biophys 197(1):57-62
59. James GT, Thach AB, Klassen L, Austin JH Studies in metachromatic leukodystrophy: XV. 1985. Purification of normal and mutant lysosomal alpha-mannosidase from human liver. Life Sci 37(25):2365-2371
60. Joss V, Rogers TR, Hugh-Jones K, Beilby B, Joshi R, Williamson S, Foroozanfar N, Riches P, Turner M, Benson PD, Hobbs JR. 1982 Exp Hematol 10, 52
61. Kelly BM, Yu CZ, Chang PL. 1989. Presence of a lysosomal enzyme, arylsulfatase-A, in the prelysosome-endosome compartments of human cultured fibroblasts. Eur J Cell Biol 48(1):71-78
62. Klein MA, Frigg R, Flechsig E, Raeber AJ, Kalinke U, Bluethmann H, Bootz F, Suter M, Zinkernagel RM, Aguzzi A. 1997. A crucial role for B cells in neuroinvasive scrapie. Nature 390(6661):687-690
63. Kreysing J, Polten A, Hess B, von Figura K, Menz K, Steiner F, Gieselmann V. 1994. Structure of the mouse lysosomal alpha-mannosidase gene and cDNA. Genomics 19(2):249-256
64. Kreysing J, von Figura K, Gieselmann V. 1990. Structure of the lysosomal alpha-mannosidase gene. Eur J Biochem 1990. Eur J Biochem. 191(3): 627-631
65. Krivit W, Shapiro E, Kennedy W, Lipton M, Lockman L, Smith S, Summers CG, Wenger DA, Tsai MY, Ramsay NK, et al. 1990. Treatment of late infantile metachromatic leukodystrophy by bone marrow transplantation. N Engl J Med 322:28-32
66. Krivit W, Shapiro E, Hoogerbrugge PM, Moser HW. 1992. State of the art review. Bone marrow transplantation treatment for storage diseases. Keystone. January 23, 1992. Bone Marrow Transplant 10 Suppl 1:87-96
67. Laidler PM, Waheed A, Van Etten RL. 1985. Structural and immunochemical characterization of human urine lysosomal alpha-mannosidase purified by affinity chromatography. Biochim Biophys Acta 827(1):73-83
68. Lee GD, van Etten RL. 1975. Evidence of an essential histidine residue in rabbit liver aryl sulfatase A. Arch Biochem Biophys 171(2):424-434
69. Luijten JAFM, Van Der Heijden MCM, Rijksen G, Staal GEJ. 1978. Purification and characterization of lysosomal alpha-mannosidase from human urine. J Mol Med. 1978, 3, 213
70. Manowitz P, Fine LV, Nora R, Chokroverty S, Nathan PE, Fazzaro JM. 1988. A new electrophoretic variant of lysosomal alpha-mannosidase. Biochem Med Metab Biol 39(1):117-120
71. Mould DL, Dawson AM, Rennie JC. 1970. Very early replication of scrapie in lymphocytic tissue. Nature 228(273):779-780
72. Ohashi T, Watabe K, Sato Y, Saito I, Barranger JA, Matalon R, Eto Y. 1996. Gene therapy for metachromatic leukodystrophy. Acta Paediatr Jpn 38(2):193-201
73. Oya Y, Nakayasu H, Fujita N, Suzuki K, Suzuki K. 1998. Pathological study of mice with total deficiency of sphingolipid activator proteins (SAP knockout mice). Acta Neuropathol (Berl) 96(1):29-40
74. Poretz RD, Yang RS, Canas B, Lackland H, Stein S, Manowitz P. 1992. The structural basis for the electrophoretic isoforms of normal and variant human platelet arylsulphatase A. Biochem J 287 (Pt 3):979-983
75. Porter MT, Fluharty AL, Kihara H. 1971. Correction of abnormal cerebroside sulfate metabolism in cultured metachromatic leukodystrophy fibroblasts. Science 1971,172, 1263-1265
76. Sangalli A, Taveggia C, Salviati A, Wrabetz L, Bordignon C, Severini GM. 1998. Transduced fibroblasts and metachromatic leukodystrophy lymphocytes transfer lysosomal alpha-mannosidase to myelinating glia and deficient cells in vitro. Hum Gene Ther 9(14):2111-2119
77. Sarafian TA, Tsay KK, Jackson WE, Fluharty AL, Kihara H. 1985. Studies on the charge isomers of lysosomal alpha-mannosidase. Biochem Med 33(3):372-380
78. Schmidt B, Selmer T, Ingendoh A, von Figura K. 1995. A novel amino acid modification in sulfatases that is defective in multiple sulfatase deficiency. Cell 82(2):271-278
79. Selmer T, Hallmann A, Schmidt B, Sumper M, von Figura K. 1996. The evolutionary conservation of a novel protein modification, the conversion of cysteine to serinesemialdehyde in arylsulfatase from Volvox carteri. Eur J Biochem 238:341-345
80. Shapira E, Nadler HL. 1975. Purification and some properties of soluble human liver arylsulfatases. Arch Biochem Biophys 170(1):179-187
81. Shapiro EG, Lockman LA, Balthazor M, Krivit W. 1995. Neuropsychological outcomes of several storage diseases with and without bone marrow transplantation. J Inherit Metab Dis 18:413-429
82. Stein C, Gieselmann V, Kreysing J, Schmidt B, Pohlmann R, Waheed A, Meyer HE, O'Brien JS, von Figura K. 1989. Cloning and expression of human lysosomal alpha-mannosidase. J Biol Chem 264:1252-1259
83. Stevens RL, Fluharty AL, Skokut MH, Kihara H. 1975. Purification and properties of arylsulfatase. A from human urine. J Biol Chem 250(7):2495-2501
84. Stevens RL, Fluharty AL, Killgrove AR, Kihara H. 1976. Microheterogeneity of lysosomal alpha-mannosidase from human tissues. Biochim Biophys Acta 445(3):661-671
85. von Figura K, Steckel F, Conary J, Hasilik A, Shaw E. 1986. Heterogeneity in late-onset metachromatic leukodystrophy. Effect of inhibitors of cysteine proteinases. Am J Hum Genet, 39, 371-382
86. Waheed A, Hasilik A, von Figura K. 1982. Synthesis and processing of lysosomal alpha-mannosidase in human skin fibroblasts. Hoppe Seylers Z Physiol Chem 363(4):425-430
87. Waheed A, van Etten RL. 1985. Phosphorylation and sulfation of lysosomal alpha-mannosidase accompanies biosynthesis of the enzyme in normal and carcinoma cell lines. Biochim Biophys Acta 847(1): 53-61
88. WO 02/99092 A (Fogh Jens; Hemebiotech AS (DK); Andersson Claes (SE); Weigelt Cecilia) 12 December 2002
89. Berg Thomas et al.: "Purification and characterization of recombinant human lysosomal alpha-mannosidase", Molecular Genetics and Metabolism, vol. 73, no. 1, April 2001, pages 18-29

## Claims

1. Use of a lysosomal alpha-mannosidase for the preparation of a medicament for treatment of alpha-mannosidosis, wherein said medicament is to be administered by intravenous administration.

2. Use according to claim 1, wherein the medicament is to be administered by
infusion, or
repeated intravenous injection in a frequency corresponding to 1 to 5 daily injections, 1 to 5 injections per week, 1 to 5 injections every 2 weeks or 1 to 5 injections every month.

3. Use according to claim 1 or claim 2, wherein said medicament does not comprise a component with a known ability to target cells within the central nervous system.

4. Use according to any one of claims 1-3, wherein the lysosomal alpha-mannosidase comprises the amino acid sequence of SEQ ID NO:1.

5. Use according to any one of claims 1-4, wherein said medicament isto be administered at a frequency corresponding to 1-5 injections per week.

6. Use according to any one of claims 1-5, wherein said medicament does not comprise a component for targeting of cells within the central nervous system and does not comprise a component with the ability to act as vehicle for the passage over the blood-brain barrier.

7. Use according to any of the preceding claims wherein said medicament is administered in an amount, which is within the range of 5 to 300 mU lysosomal alpha-mannosidase per gram body weight of the subject to be treated.

8. Use according to any of the preceding claims, wherein said medicament is administered in an amount, which is within the range of 20 to 100 mU lysosomal alpha-mannosidase per gram body weight of the subject to be treated.

9. Use according to any of the preceding claims, wherein a fraction of said lysosomal alpha-mannosidase consists of lysosomal alpha-mannosidase having one or more N-linked oligosaccharides carrying mannose 6-phosphate residues.

10. Use according to any of the preceding claims, wherein a fraction corresponding to from 1 to 75% of the activity of a preparation of lysosomal alpha-mannosidase binds to the mannose-6-phosphate-receptor.

11. Use according to claim 10, wherein said fraction corresponds to from 10 to 50% of the activity of a preparation of lysosomal alpha-mannosidase.

12. Use according to claim 10, wherein said fraction corresponds to from 20 to 40% of the activity of a preparation of lysosomal alpha-mannosidase.

13. Use according to any of the preceding claims, wherein said lysosomal alpha-mannosidase is a recombinant enzyme.

14. Use according to any of the preceding claims, wherein said lysosomal alpha-mannosidase is produced in a mammalian cell system.

15. Use according to claim 14, wherein said mammalian cell system is a CHO cell line.

## Patentansprüche

1. Verwendung von lysosomaler Alpha-Mannosidase zur Herstellung eines Arzneimittels zur Behandlung von Alpha-Mannosidose, wobei das Arzneimittel durch intravenöse Injektion zu verabreichen ist.

2. Verwendung nach Anspruch 1, wobei das Arzneimittel durch
Infusion oder
wiederholte intravenöse Injektion mit einer Häufigkeit von 1 bis 5 Injektionen pro Tag, 1 bis 5 Injektionen pro Woche, 1 bis 5 Injektionen alle 2 Wochen oder 1 bis 5 Injektionen pro Monat zu verabreichen ist.

3. Verwendung nach Anspruch 1 oder Anspruch 2, wobei das Arzneimittel keine Komponente, die in der Lage ist, auf Zellen innerhalb des zentralen Nervensystems gerichtet zu sein, umfasst.

4. Verwendung nach einem der Ansprüche 1-3, wobei die lysosomale Alpha-Mannosidase die Aminosäuresequenz von SEQ ID NR.: 1 umfasst.

5. Verwendung nach einem der Ansprüche 1-4, wobei das Arzneimittel mit einer Häufigkeit von 1-5 Injektionen pro Woche zu verabreichen ist.

6. Verwendung nach einem der Ansprüche 1-5, wobei das Arzneimittel keine Komponente, die auf Zellen innerhalb des zentralen Nervensystems gerichtet ist, und keine Komponente, die in der Lage ist, als Träger zum Passieren der Blut-Hirn-Schranke zu dienen, umfasst.

7. Verwendung nach einem der vorhergehenden Ansprüche, wobei das Arzneimittel in einer Menge verabreicht wird, die im Bereich von 5 bis 300 mU lysosomale Alpha-Mannosidase pro Gramm Körpergewicht des zu behandelnden Patienten liegt.

8. Verwendung nach einem der vorhergehenden Ansprüche, wobei das Arzneimittel in einer Menge verabreicht wird, die im Bereich von 20 bis 100 mU lysosomale Alpha-Mannosidase pro Gramm Körpergewicht des zu behandelnden Patienten liegt.

9. Verwendung nach einem der vorhergehenden Ansprüche, wobei eine Fraktion der lysosomalen Alpha-Mannosidase aus lysosomaler Alpha-Mannosidase mit einem oder mehreren Mannose-6-Phosphat-Reste tragenden N-verbundenen Oligosacchariden besteht.

10. Verwendung nach einem der vorhergehenden Ansprüche, wobei eine Fraktion, die 1 bis 75 % der Aktivität einer Zubereitung aus lysosomaler Alpha-Mannosidase entspricht, an den Mannose-6-Phosphat-Rezeptor bindet.

11. Verwendung nach Anspruch 10, wobei die Fraktion 10 bis 50 % der Aktivität einer Zubereitung aus lysosomaler Alpha-Mannosidase entspricht.

12. Verwendung nach Anspruch 10, wobei die Fraktion 20 bis 40% der Aktivität einer Zubereitung aus lysosomaler Alpha-Mannosidase entspricht.

13. Verwendung nach einem der vorhergehenden Ansprüche, wobei die lysosomale Alpha-Mannosidase ein rekombinantes Enzym ist.

14. Verwendung nach einem der vorhergehenden Ansprüche, wobei die lysosomale Alpha-Mannosidase in einem Säugetierzellsystem produziert ist.

15. Verwendung nach Anspruch 14, wobei das Säugetierzellsystem eine CHO-Zelllinie ist.

## Revendications

1. Utilisation d'une alpha-mannosidase lysosomale pour la préparation d'un médicament pour le traitement de l'alpha-mannosidose, dans laquelle ledit médicament doit être administré par administration intraveineuse.

2. Utilisation selon la revendication 1, dans laquelle ledit médicament doit être administré
par perfusion ou
par injections intraveineuses successives à une fréquence de 1 à 5 injections par jour, 1 à 5 injections par semaine, 1 à 5 injections toutes les 2 semaines ou 1 à 5 injections par mois.

3. Utilisation selon la revendication 1 ou 2, ledit médicament ne comprenant pas de composant présentant une aptitude particulière de cibler des cellules au sein du système nerveux central.

4. Utilisation selon l'une quelconque des revendications 1 à 3, dans laquelle l'alpha-mannosidase lysosomale comprend la séquence d'acides aminés de SEQ ID NO:1.

5. Utilisation selon l'un quelconque des revendications 1 à 4, dans laquelle ledit médicament doit être administré à une fréquence correspondant à 1 à 5 injections par semaine.

6. Utilisation selon l'une quelconque des revendications 1 à 5, dans laquelle ledit médicament ne comprend pas de composant pour cibler des cellules du système nerveux central et ne comprend pas de composant capable d'agir comme véhicule pour le passage de la barrière hémato-encéphalique.

7. Utilisation selon l'une quelconque des revendications précédentes, dans laquelle ledit médicament est administré en une quantité qui se situe dans la plage de 5 à 300 mU d'alpha-mannosidase lysosomale par gramme de poids corporel du sujet à traiter.

8. Utilisation selon l'une quelconque des revendications précédentes, dans laquelle ledit médicament est administré en une quantité qui se situe dans la plage de 20 à 100 mU d'alpha-mannosidase lysosomale par gramme de poids corporel du sujet à traiter.

9. Utilisation selon l'une quelconque des revendications précédentes, dans laquelle une fraction de ladite alpha-mannosidase lysosomale se compose d'alpha-mannosidase lysosomale possédant un ou plusieurs oligosaccharides liés à N porteurs de résidus de mannose 6-phosphate.

10. Utilisation selon l'une quelconque des revendications précédentes, dans laquelle une fraction correspondant à 1 à 75 % de l'activité d'une préparation d'alpha-mannosidase lysosomale se lie au récepteur du mannose 6-phosphate.

11. Utilisation selon la revendication 10, dans laquelle ladite fraction correspond à 10 à 50 % de l'activité d'une préparation d'alpha-mannosidase lysosomale.

12. Utilisation selon la revendication 10, dans laquelle ladite fraction correspond à 20 à 40 % de l'activité d'une préparation d'alpha-mannosidase lysosomale.

13. Utilisation selon l'une quelconque des revendications précédentes, dans laquelle ladite alpha-mannosidase lysosomale est une enzyme recombinante.

14. Utilisation selon l'une quelconque des revendications précédentes, dans laquelle ladite alpha-mannosidase lysosomale est produite dans un système cellulaire de mammifère.

15. Utilisation selon la revendication 14, dans laquelle ledit système cellulaire de mammifère est une lignée cellulaire CHO.
